# EUROPEAN PATENT APPLICATION

(11) **EP 4 144 505 A1**
(43) Date of publication of application: **08.03.2023**
(21) Application number: 21796888.2
(22) Date of filing: 28.04.2021
(51) Int. Cl.: B29C 64/112, B29C 64/209, B29C 64/336, B33Y 30/00, B33Y 50/02

(54) **IN VIVO VESSEL REPAIR DEVICE AND OPERATION METHOD THEREFOR**

(30) Priority: 30.04.2020 CN 202010365786
(71) Applicant: Revotek Co., Ltd, Sichuan 611731 (CN)
(72) Inventor: ZUO, Xiao, Chengdu, Sichuan 611731 (CN); HE, Junxuan, Chengdu, Sichuan 611731 (CN); LI, Yijun, Chengdu, Sichuan 611731 (CN); XIANG, Jie, Chengdu, Sichuan 611731 (CN); XU, Ziqing, Chengdu, Sichuan 611731 (CN); JIANG, Zhi, Chengdu, Sichuan 611731 (CN)
(74) Representative: Kolster Oy Ab
(86) International application number: PCT/CN2021/090603
(87) International publication number: WO 2021/219011

(57) **Abstract**

Disclosed are an in vivo vessel repair device and an operation method therefor. The in vivo vessel repair device comprises: a catheter (20); a spray head assembly, comprising a supply end (10B) and a spraying end (10A) at two axial ends thereof, and having a first material flow channel (P1), a first material nozzle (J1), a second material flow channel (P2) and a second material nozzle (J2), with the first material flow channel (P1) being arranged inside the spray head assembly, the first material nozzle (J1) being in communication with the first material flow channel (P1) and spraying a first material onto the radial outer side, the second material flow channel (P2) being arranged inside the spray head assembly and isolated from the first material flow channel (P1), the second material nozzle (J2) being in communication with the second material flow channel (P2) and spraying a second material onto the radial outer side, and the spray head assembly being configured to enter the catheter (20) and extend out of the spraying end (10A) from a leading-in end (20A) of the catheter (20); and a driving device (40) configured to be connected to the supply end (10B) of the spray head assembly and configured to drive the spray head assembly to spray the second material and the first material. According to the device and the method, the inner wall of an in vivo vessel can be coated with a medical adhesive layer and a biological ink layer.

## Description

### Cross-Reference to Related Applications

This disclosure is based on and claims priority to Chinese Patent Application No. 202010365786.2, filed on April 30, 2020, entitled "In Vivo Duct Repair Device and Operating Method of In Vivo Duct Repair Device", which is hereby incorporated by reference in its entirety.

### Field of the Invention

The present disclosure relates to the field of bioprinting technology, in particular to an in vivo duct repair device and an operating method of the in vivo duct repair device.

### Background of the Invention

Application No. 201080017353.2, entitled "Methods and Device for Venous Occlusion for Treatment of Venous Insufficiency", discloses a vein closure device, which includes three components: a first component which is an outer catheter or introducer sheath that allows for placement under precise ultrasound guidance into the saphenous vein from as low a position as possible in the greater saphenous vein or lesser saphenous vein; a second component which is an introducer or inner catheter for a vein-occluding substance or adhesive; and a third component which is a glue gun or other adhesive introducing device that attaches to the inner catheter. The device described above releases the vein-occluding substance (medical adhesive) at corresponding locations in the vein to achieve occlusion of the vein such as the greater saphenous vein.

### Summary of the Invention

A first aspect of the present disclosure provides an in vivo duct repair device, including:
a catheter comprising an introducing end configured to be inserted into an in vivo duct;
a nozzle assembly, two axial ends of the nozzle assembly including a supplying end and a spraying end, respectively, the nozzle assembly having a first material flow channel, a first material spout, a second material flow channel and a second material spout, the first material flow channel being arranged inside the nozzle assembly, the first material spout being communicated with the first material flow channel and being configured to spray a first material radially outwards of the nozzle assembly, the second material flow channel being arranged inside the nozzle assembly and being isolated from the first material flow channel, the second material spout being communicated with the second material flow channel and being configured to spray a second material radially outwards of the nozzle assembly, and the nozzle assembly being configured to enter the catheter and extend the spraying end out of the introducing end of the catheter to spray the first material and the second material to an inner wall of the in vivo duct; and
a drive device configured to be connected to the supplying end of the nozzle assembly and configured to drive the nozzle assembly to spray the first material and the second material, wherein one of the first material and the second material is bio-ink, and the other of the first material and the second material is medical adhesive.

In some embodiments,
the first material spout is located on a side face of the spraying end; and/or the second material spout is located on a side face of the spraying end.

In some embodiments, the second material spout is arranged staggeredly with respect to the first material spout in an axial direction of the nozzle assembly.

In some embodiments, the first material spout is closer to an end face of the spraying end in the axial direction of the nozzle assembly than the second material spout, wherein the first material spout is configured to spray the bio-ink, and the second material spout is configured to spray the medical adhesive.

In some embodiments,
the nozzle assembly includes a plurality of first material spouts uniformly distributed circumferentially of the nozzle assembly; and/or
the nozzle assembly includes a plurality of second material spouts uniformly distributed circumferentially of the nozzle assembly or the second material spout includes an annular spout arranged around the axial direction of the nozzle assembly.

In some embodiments, the nozzle assembly includes a medical adhesive scrape coating part arranged on a side face of the nozzle assembly, the medical adhesive scrape coating part being located between the first material spout and the second material spout in the axial direction of the nozzle assembly.

In some embodiments, the medical adhesive scrape coating part includes an annular projection or brush arranged on a side face of the nozzle assembly.

In some embodiments, the nozzle assembly includes:
a first nozzle body, the first material spout being arranged on the first nozzle body; and
a second nozzle body arranged at the outer periphery of the first nozzle body, the second material spout being arranged on the second nozzle body, or the second material spout being arranged between the first nozzle body and the second nozzle body.

In some embodiments,
the first material flow channel is arranged in the first nozzle body; and
the second material flow channel is arranged between the second nozzle body and the first nozzle body.

In some embodiments, the nozzle assembly includes a connecting piece, which is connected between the first nozzle body and the second nozzle body.

In some embodiments,
the first material flow channel is arranged in the first nozzle body;
the second material flow channel is arranged in the first nozzle body, a side wall of which has a communication port communicated with the second material flow channel; and
the second material spout includes an annular spout arranged around the axial direction of the nozzle assembly, formed by the first nozzle body and an end of the second nozzle body close to the spraying end, the communication port being communicated with the annular spout.

In some embodiments, the nozzle assembly further includes a nozzle mounting seat, wherein
the first material flow channel includes a first material nozzle flow channel arranged in the first nozzle body and a first material mounting seat flow channel arranged in the nozzle mounting seat, or the first material flow channel is arranged in the nozzle mounting seat; and
the second material flow channel includes a second material nozzle flow channel arranged in the second nozzle body and a second material mounting seat flow channel arranged in the nozzle mounting seat.

In some embodiments, the second material flow channel further includes a first annular buffer groove, the second material nozzle flow channel and the second material mounting seat flow channel being communicated through the first annular buffer groove.

In some embodiments, the first annular buffer groove is formed between the second nozzle body and the nozzle mounting seat.

In some embodiments, the second material flow channel includes a second annular buffer groove arranged between the second material spout and the second material nozzle flow channel.

In some embodiments, at least one of the first material spout and the second material spout is arranged on the end face of the spraying end.

In some embodiments, the in vivo duct repair device further includes a guide wire configured to obtain an initial passageway within the in vivo duct, and the catheter is inserted along the guide wire into the in vivo duct.

In some embodiments, the drive device includes:
a first material supply part in communication with the first material flow channel to supply the first material to the first material flow channel; and/or
a second material supply part in communication with the second material flow channel to supply the second material to the second material flow channel.

In some embodiments,
the nozzle assembly includes a first material storage part, and the drive device includes a first drive part, an outlet of the first material storage part being communicated with the first material flow channel, the first drive part being coupled with the first material storage part to drive the first material storage part to output the first material from the outlet thereof; and/or
the nozzle assembly includes a second material storage part, and the drive device includes a second drive part, an outlet of the second material storage part being communicated with the second material flow channel, the second drive part being coupled with the second material storage part to drive the second material storage part to output the second material from the outlet thereof.

In some embodiments,
the first material storage part includes a first bag storing the first material, and the first drive part includes a first fluid pump; and/or
the second material storage part includes a second bag storing the second material, and the second drive part includes a second fluid pump.

In some embodiments,
the first bag is located in the first material flow channel and separates the first material flow channel into an upstream segment and a downstream segment, the first fluid pump being communicated with the upstream segment of the first material flow channel, an outlet of the first bag being communicated with the downstream segment of the first material flow channel; and/or
the second bag is located in the second material flow channel and separates the second material flow channel into an upstream segment and a downstream segment, the second fluid pump being communicated with the upstream segment of the second material flow channel, and an outlet of the second bag being communicated with the downstream segment of the second material flow channel.

A second aspect of the present disclosure provides an operating method of the in vivo duct repair device in the first aspect of the present disclosure, the method including:
inserting the introducing end of the catheter into a to-be-repaired area in the in vivo duct;
extending the nozzle assembly into the catheter and extending the spraying end of the nozzle assembly out of the introducing end of the catheter; and
driving, by the drive device, the nozzle assembly to spray the first material and the second material to the to-be-repaired area to form a medical adhesive layer on the to-be-repaired area and to form a bio-ink layer on the medical adhesive layer.

The present disclosure can achieve the coating of the medical adhesive and the bio-ink on the inner wall of the in vivo duct and the formation of a double-layer structure with a medical adhesive layer and a bio-ink layer, thereby achieving repair of a damaged part of the inner wall of the in the in vivo duct.

Other features and advantages of the present disclosure will become apparent from the following detailed description of exemplary embodiments of the present disclosure with reference to the accompanying drawings.

### Brief Description of the Drawings

Drawings illustrated herein are used for providing further understanding of the present disclosure and form part of the present application, and illustrative embodiments of the present disclosure and description thereof are intended for explaining instead of improperly limiting the present disclosure. In the drawings:
Fig. 1 is a schematic structural diagram of an in vivo duct repair device of an embodiment of the present disclosure.
Fig. 2 is a three-dimensional structural diagram of a nozzle assembly of an in vivo duct repair device of an embodiment of the present disclosure.
Fig. 3 is a sectional structural diagram of the nozzle assembly of the embodiment shown in Fig. 2 at an angle.
Fig. 4 is a sectional structural diagram of the nozzle assembly of the embodiment shown in Fig. 2 at another angle.
Fig. 5 is a sectional structural diagram of Fig. 4 at A-A.
Fig. 6 is a three-dimensional structural diagram of a nozzle assembly of an in vivo duct repair device of an embodiment of the present disclosure.
Fig. 7 is a sectional structural diagram of the nozzle assembly of the embodiment shown in Fig. 6.
Fig. 8 is a three-dimensional structural diagram of a nozzle assembly of an in vivo duct repair device of an embodiment of the present disclosure.
Fig. 9 is a sectional structural diagram of the nozzle assembly of the embodiment shown in Fig. 8.
Fig. 10 is a three-dimensional structural diagram of a first nozzle body of the nozzle assembly of the embodiment shown in Fig. 8.
Fig. 11 is a sectional structural diagram of the first nozzle body of Fig. 10.
Fig. 12 is three-dimensional structural diagram of a second nozzle body of the nozzle assembly of the embodiment shown in Fig. 8.
Fig. 13 is a sectional structural diagram of the second nozzle body of Fig. 12.
Fig. 14 is a three-dimensional structural diagram of a nozzle mounting seat of the nozzle assembly of the embodiment shown in Fig. 8.
Fig. 15 is a sectional structural diagram of the nozzle mounting seat of Fig. 14.
Fig. 16 is a top-view structural diagram of the nozzle mounting seat of Fig. 14.
Fig. 17 is a three-dimensional structural diagram of a nozzle assembly of an in vivo duct repair device of an embodiment of the present disclosure.
Fig. 18 is a sectional structural diagram of the nozzle assembly of the embodiment shown in Fig. 17.
Fig. 19 is a three-dimensional structural diagram of a first nozzle body of the nozzle assembly of the embodiment shown in Fig. 17.
Fig. 20 is a sectional structural diagram of the first nozzle body of Fig. 19.
Fig. 21 is three-dimensional structural diagram of a second nozzle body of the nozzle assembly of the embodiment shown in Fig. 17.
Fig. 22 is a sectional structural diagram of the second nozzle body of Fig. 21.
Fig. 23 is a three-dimensional structural diagram of a nozzle mounting seat of the nozzle assembly of the embodiment shown in Fig. 17.
Fig. 24 is a sectional structural diagram of the nozzle mounting seat of Fig. 23.
Fig. 25 is a structural diagram of an in vivo duct to be repaired.
Fig. 26 is a schematic structural diagram illustrating an introducing end of a catheter is inserted into the in vivo duct.
Fig. 27 is a schematic structural diagram illustrating a spraying end of the nozzle assembly is extended from the introducing end of the catheter to an initial repair position.
Fig. 28 is a schematic structural diagram illustrating the spraying end of the nozzle assembly starts coating.
Fig. 29 is a structural diagram of the in vivo duct that has been repaired.
Fig. 30 is a schematic structural diagram of an in vivo duct repair device of an embodiment of the present disclosure.
Fig. 31 is a schematic structural diagram of an in vivo duct repair device of an embodiment of the present disclosure.
Fig. 32 is a three-dimensional structural diagram of a nozzle assembly of an in vivo duct repair device of an embodiment of the present disclosure.
Fig. 33 is a three-dimensional structural diagram of a nozzle assembly of an in vivo duct repair device of an embodiment of the present disclosure.
Fig. 34 is a three-dimensional structural diagram of a nozzle assembly of an in vivo duct repair device of an embodiment of the present disclosure.
Fig. 35 is a three-dimensional structural diagram of a nozzle assembly of an in vivo duct repair device of an embodiment of the present disclosure.
Fig. 36 is a schematic structural diagram of an in vivo duct repair device of an embodiment of the present disclosure in use when the spraying end of the nozzle assembly is extended out of the introducing end of the catheter and arrives at a second end of a to-be-repaired area of the in vivo duct.
Fig. 37 is a schematic structural diagram of the in vivo duct repair device of the embodiment shown in Fig. 36 in use when the spraying end of the nozzle assembly is extended out of the introducing end of the catheter and continues moving toward a first end of the to-be-repaired area.
Fig. 38 is a schematic structural diagram of the in vivo duct repair device of the embodiment shown in Fig. 36 in use when the spraying end of the nozzle assembly starts moving from the first end of the to-be-repaired area toward the second end of the to-be-repaired area and starts spraying repair materials.
Fig. 39 is a schematic structural diagram of the arrangement of a first material spout and a second material spout at the spraying end of the nozzle assembly of the in vivo duct repair device of the embodiment shown in Fig. 36.
Fig. 40 is a schematic structural diagram of an in vivo duct repair device of an embodiment of the present disclosure in use when the spraying end of the nozzle assembly is extended out of the introducing end of the catheter and arrives at a second end of a to-be-repaired area of the in vivo duct.
Fig. 41 is a schematic structural diagram of the in vivo duct repair device of the embodiment shown in Fig. 40 in use when the spraying end of the nozzle assembly is extended out of the introducing end of the catheter and continues moving toward a first end of the to-be-repaired area.
Fig. 42 is a schematic structural diagram of the in vivo duct repair device of the embodiment shown in Fig. 40 in use when the spraying end of the nozzle assembly starts moving from the first end of the to-be-repaired area toward the second end of the to-be-repaired area and starts spraying repair materials.
Fig. 43 is a schematic structural diagram of the arrangement of a first material spout and a second material spout at the spraying end of a nozzle assembly of the in vivo duct repair device of the embodiment shown in Fig. 40.
Fig. 44 is a schematic structural diagram of the arrangement of a first material spout and a second material spout at the spraying end of another nozzle assembly of the in vivo duct repair device of the embodiment shown in Fig. 40.

### Detailed Description of the Embodiments

Technical solutions in the embodiments will be described below clearly and completely in conjunction with the accompanying drawings in the embodiments of the present disclosure. Obviously, the described embodiments are only a part of the embodiments of the present application, and not all the embodiments. The following description of at least one exemplary embodiment is actually only illustrative, and in no way serves as any limitation on the present disclosure and its application or use. Based on the embodiments in the present disclosure, all of other embodiments obtained by a person of ordinary skill in the art without creative work should fall into the protection scope of the present disclosure.

Unless specifically stated otherwise, the relative arrangement of components and steps, numerical expressions and numerical values set forth in these embodiments do not limit the scope of the present disclosure. Furthermore, it should be appreciated that, for ease of description, the sizes of various parts shown in the drawings are not drawn in accordance with actual proportional relationships. Technologies, methods, and devices known to those of ordinary skill in the related art may be not discussed in detail, but where appropriate, the technologies, methods, and device should be regarded as part of the specification as granted. In all examples shown and discussed here, any specific value should be interpreted as merely exemplary, rather than as a limitation. Therefore, other examples of an exemplary embodiment may have different values. It should be noted that similar reference numerals and letters denote similar items in the following drawings, so once a certain item is defined in one drawing, it does not need to be further discussed in subsequent drawings.

In the description of the present disclosure, it should be understood that the use of terms such as "first" and "second" to define parts and components is only for the convenience of distinguishing the corresponding parts and components. Unless otherwise stated, the above terms have no special meanings, and therefore cannot be construed as limitations on the protection scope of the present disclosure.

In the process of implementing the present disclosure, the inventors found that the technical solutions disclosed in the related technologies in the foregoing background art achieve closure of damaged lumens, but cannot achieve repair of damaged parts of in vivo ducts.

The so-called in vivo ducts in the present disclosure mainly refer to in vivo ducts in living creatures, including human in vivo ducts (e.g. including human blood vessels, bile ducts, esophagus, intestinal tracts, respiratory tracts, etc.) and animal in vivo ducts (e.g. blood vessels, bile ducts, intestine tracts, etc. in pigs).

To achieve repair of damaged parts of in vivo ducts, embodiments of the present disclosure provide an in vivo duct repair device and an operating method of the in vivo duct repair device.

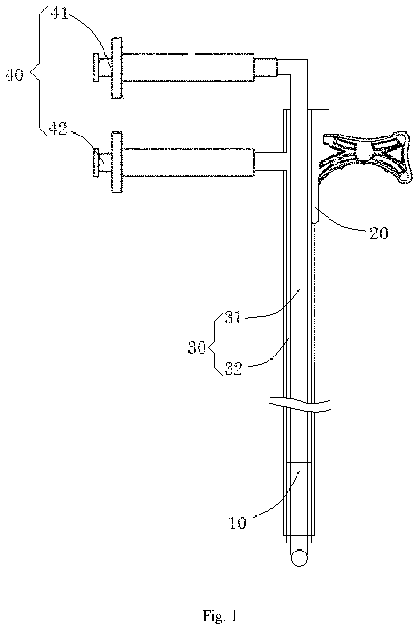

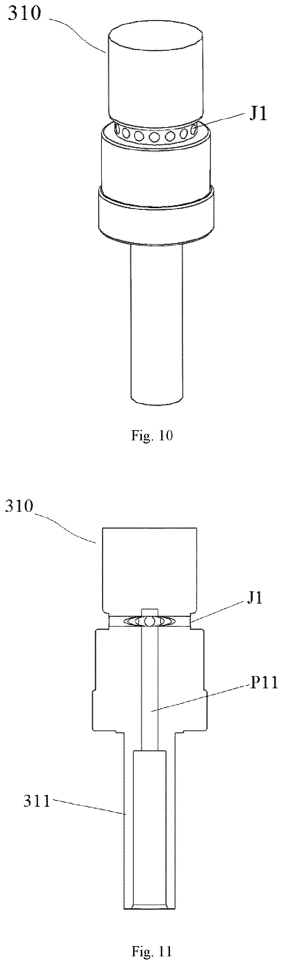

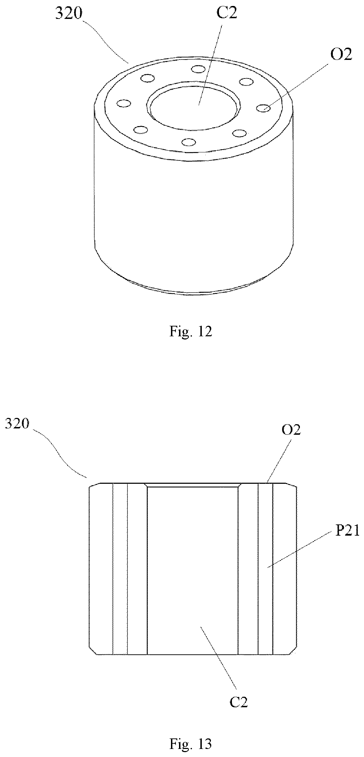

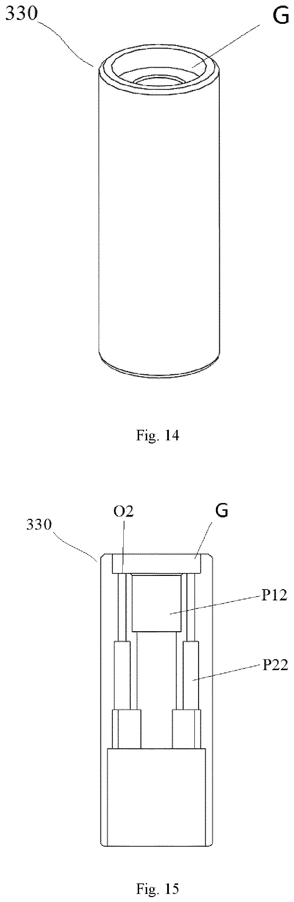

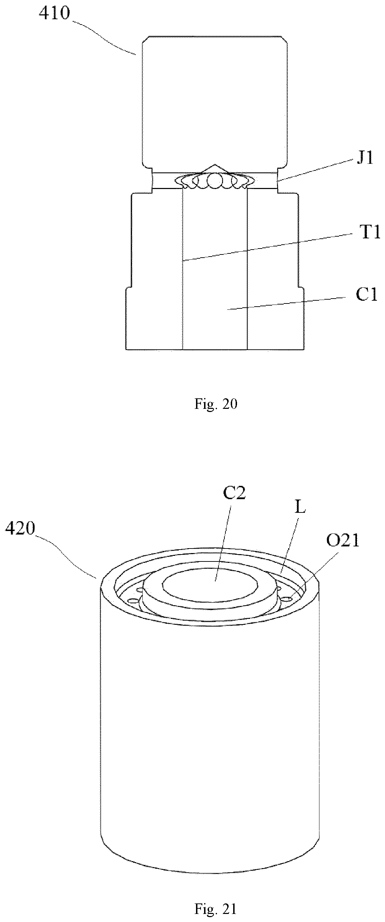

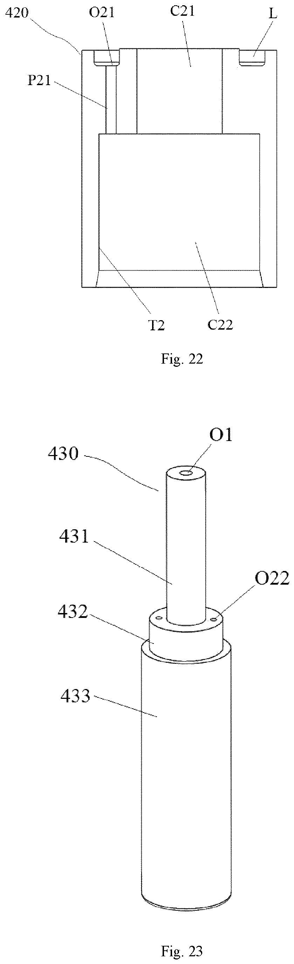

As shown in Figs. 1 to 35, the in vivo duct repair device of the embodiments of the present disclosure includes a catheter 20, a nozzle assembly and a drive device 40. The catheter 20 includes an introducing end 20A configured to be inserted into an in vivo duct 90, and two axial ends of the nozzle assembly include a supplying end 10B and a spraying end 10A, respectively. The nozzle assembly has a first material flow channel P1, a first material spout J1, a second material flow channel P2 and a second material spout J2. The first material flow channel P1 is arranged inside the nozzle assembly. The first material spout J1 is communicated with the first material flow channel P1 and is configured to spray a first material radially outwards of the nozzle assembly; the second material flow channel P2 is arranged inside the nozzle assembly and is isolated from the first material flow channel P1; the second material spout J2 is communicated with the second material flow channel P2 and is configured to spray a second material radially outwards of the nozzle assembly; and the nozzle assembly is configured to enter the catheter 20 and extend the spraying end 10A out of the introducing end 20A of the catheter 20 to spray the first material and the second material to an inner wall of the in vivo duct 90. The drive device 40 is configured to be connected to the supplying end 10B of the nozzle assembly and is configured to drive the nozzle assembly to spray the first material and the second material. One of the first material and the second material is bio-ink, and the other of the first material and the second material is medical adhesive.

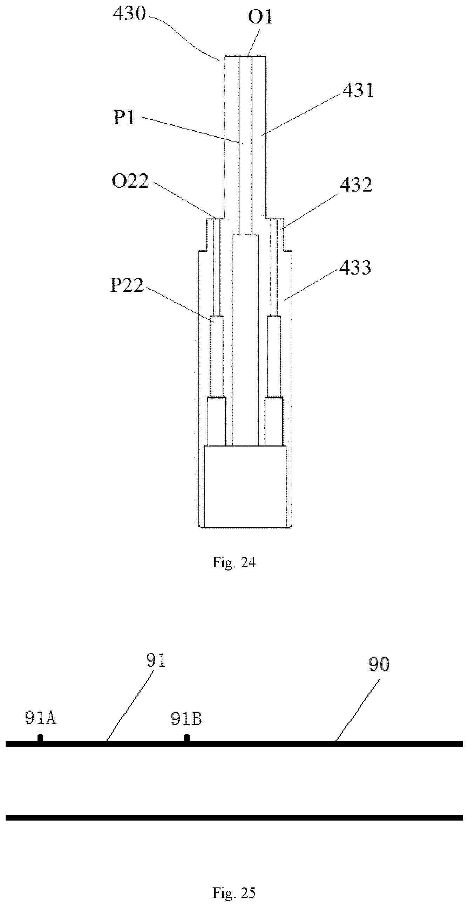

The present disclosure can achieve the coating of the medical adhesive and the bio-ink on the inner wall of the in vivo duct and the formation of a double-layer structure with a medical adhesive layer and a bio-ink layer. The in vivo duct repair device of the embodiments of the present disclosure can achieve the coating of the medical adhesive and the bio-ink on the inner wall of the in vivo duct 90. In conjunction with Figs. 25 to 29, to repair a to-be-repaired area 91 of the in vivo duct 90, the introducing end 20A of the catheter 20 can be first inserted into the to-be-repaired area 91 of the in vivo duct 90, and then the nozzle assembly is extended into the catheter 20 such that the spraying end 10A is extended out of the introducing end 20A; the nozzle assembly includes the first material spout J1 and the second material spout J2, which can spray the first material and the second material to the inner wall of the in vivo duct 90 such that the to-be-repaired area 91 of the in vivo duct 90 is coated with the medical adhesive and the bio-ink successively, so as to directly form a double-layer structure with a medical adhesive layer 91D and a bio-ink layer 91C at a damaged part of the in vivo duct 90, and achieve repair of the damaged part of the in vivo duct 90.

In the in vivo duct repair device of some embodiments, the first material spout J1 is located on a side face of the spraying end; and/or the second material spout J2 is located on a side face of the spraying end. Providing the first material spout J1 and/or the second material spout J2 on a side face of the spraying end of the nozzle assembly facilitates better contact of the sprayed repair materials with the inner wall of the in vivo duct.

In the in vivo duct repair device of some embodiments, the second material spout J2 is arranged staggeredly with respect to the first material spout J1 in an axial direction of the nozzle assembly.

With the first material spout J1 and the second material spout J2 arranged staggeredly in the axial direction of the nozzle assembly, the nozzle assembly 10 can move in the catheter 20 along the in vivo duct 90 when repairing the in vivo duct 90, such that the medical adhesive is sprayed first, and as the nozzle assembly 10 moves relative to the in vivo duct 90, the medical adhesive is coated on the inner wall of the in vivo duct 90 to form the medical adhesive layer 91D. The bio-ink is sprayed at short time after the medical adhesive is sprayed. Due to an adhering effect of the medical adhesive, the bio-ink can be adhered to the medical adhesive layer 91D immediately after being sprayed, to ensure the formation of the medical adhesive layer 91D and the bio-ink layer 91C at the same time on the inner wall of the in vivo duct 90 to form the desired double-layer structure.

In the in vivo duct repair device of some embodiments, the first material spout J1 is closer to an end face of the spraying end 10A in the axial direction of the nozzle assembly 10 than the second material spout J2, wherein the first material spout J1 is configured to spray the bio-ink, and the second material spout J2 is configured to spray the medical adhesive. When the in vivo duct 90 is repaired by using the nozzle assembly of this structure, since the bio-ink is supplied to the first material spout J1 and the medical adhesive is supplied to the second material spout J2 by the drive assembly 40, so that the bio-ink can be sprayed from the first material spout J1 and the medical adhesive can be sprayed from the second material spout J2, thus when coating begins, the drive assembly 40 drives both the bio-ink and the medical adhesive to be supplied, and since the positions of the first material spout J1 and the second material spout J2 are staggered, and the first material spout J1 is closer to the end face of the spraying end 10A, the medical adhesive, when being sprayed, comes into contact with the inner wall of the in vivo duct 90 before the bio-ink, and as the nozzle assembly 10 moves from a first end 91A of the to-be-repaired area 91 of the in vivo duct 90 away from toward a second end 91B, the medical adhesive is coated on the inner wall of the in vivo duct 90, and the medical adhesive layer can be formed on the inner wall of the in vivo duct 90 due to the adhering effect of the medical adhesive; with the movement of the nozzle assembly 10, the bio-ink comes into contact with the medical adhesive layer at short time after the medical adhesive comes into contact with the inner wall of the in vivo duct 90, and at the same time the bio-ink can be adhered to the medical adhesive layer due to the adhering effect of the medical adhesive; and with the continuous movement of the nozzle assembly 10, the bio-ink is coated on the medical adhesive layer, to form the bio-ink layer on the medical adhesive layer, thus ensuring the formation of the medical adhesive layer and the bio-ink layer on the inner wall of the in vivo duct 90. The nozzle assembly 10 can be extended from the introducing end 20A of the catheter 20 to a position in the to-be-repaired area 91 farthest from the introducing end 20A, and simultaneous formation of the coated medical adhesive layer 91D and bio-ink layer 91C on the inner wall of the in vivo duct 90 is achieved in a process of moving from the farthest position to a proximal position; moreover, since the above structure can achieve simultaneous coating of the medical adhesive and the bio-ink by moving once, the nozzle assembly 10 gradually moves from the farthest position to the introducing end 20A in the coating process, thereby facilitating the nozzle assembly in the in vivo duct 90 returning to the introducing end 20A of the catheter 20 while coating during the repair of the in vivo duct 90, and the nozzle assembly can be back in the introducing end 20A when the coating is completed. Therefore, after the coating is finished, the coated bio-ink layer 91C is not affected by the returning of the nozzle assembly 10.

In the in vivo duct repair device of some embodiments, the nozzle assembly 10 includes a plurality of first material spouts J1 uniformly distributed circumferentially of the nozzle assembly; and/or the nozzle assembly includes a plurality of second material spouts J2 uniformly distributed circumferentially of the nozzle assembly or the second material spout J2 includes an annular spout arranged around the axial direction of the nozzle assembly. The above arrangement of the first material spout J1 and the second material spout J2 is conducive to uniform coating of the first material and the second material as coating materials.

In the in vivo duct repair device of some embodiments, the nozzle assembly includes a medical adhesive scrape coating part M arranged on a side face of the nozzle assembly, the medical adhesive scrape coating part M being located between the first material spout J1 and the second material spout J2 in the axial direction of the nozzle assembly.

In the in vivo duct repair device of some embodiments, the medical adhesive scrape coating part M includes an annular projection or brush arranged on a side face of the nozzle assembly.

The medical adhesive scrape coating part M is conducive to uniform coating of the medical adhesive, thereby facilitating uniform and firm adhering of the bio-ink to the inner wall of the in vivo duct 90.

In the in vivo duct repair device of some embodiments, the in vivo duct repair device further includes a guide wire configured to obtain an initial passageway within the in vivo duct 90, and the catheter 20 is inserted along the guide wire 50 into the in vivo duct 90.

The in vivo duct repair device including the guide wire 50 can achieve better guiding of the catheter 20 into the in vivo duct 90.

In the in vivo duct repair device of some embodiments, the drive device 40 includes: a first material supply part 41 in communication with the first material flow channel P1 to supply the first material to the first material flow channel P1; and/or a second material supply part 42 in communication with the second material flow channel P2 to supply the second material to the second material flow channel P2.

The first material supply part 41 is configured to supply the first material directly to the first material flow channel P1, and the second material supply part 42 is configured to supply the second material directly to the second material flow channel P2. The first material supply part 41 may be, for example, a first injection device storing the first material; and the second material supply part 42 may be, for example, a second injection device storing the second material.

In the in vivo duct repair device of some embodiments, the nozzle assembly includes a first material storage part, and the drive device 40 includes a first drive part 41A, an outlet of the first material storage part being communicated with the first material flow channel P1, the first drive part 41A being coupled with the first material storage part to drive the first material storage part to output the first material from the outlet thereof; and/or the nozzle assembly includes, and the drive device 40 includes a second drive part 41B, an outlet of the second material storage part being communicated with the second material flow channel P2, the second drive part 41B being coupled with the second material storage part to drive the second material storage part to output the second material from the outlet thereof.

With this configuration, the nozzle assembly does not need supply of the coating materials from the outside, and the drive device 40 can provide only drive forces for driving the first material storage part and the second material storage part to supply the corresponding coating materials.

In the in vivo duct repair device of some embodiments, the first material storage part includes a first bag B1 storing the first material, and the first drive part 41A includes a first fluid pump; and/or the second material storage part includes a second bag B2 storing the second material, and the second drive part 41B includes a second fluid pump. The first fluid pump is, for example, an air pump or a liquid pump, and the second fluid pump is, for example, an air pump or a liquid pump. The first fluid pump is, for example, a third injection device, and the second fluid pump is, for example, a fourth injection device.

Storing the coating materials by means of the storage bags facilitates release of the coating materials by fluid driving of the storage bags.

In the in vivo duct repair device of some embodiments, the first bag B1 is located in the first material flow channel P1 and separates the first material flow channel P1 into an upstream segment and a downstream segment, the first fluid pump being communicated with the upstream segment of the first material flow channel P1, an outlet of the first bag B1 being communicated with the downstream segment of the first material flow channel P1; and/or the second bag B2 is located in the second material flow channel P2 and separates the second material flow channel P2 into an upstream segment and a downstream segment, the second fluid pump being communicated with the upstream segment of the second material flow channel P2, and an outlet of the second bag B2 being communicated with the downstream segment of the second material flow channel P2.

Embodiments of the present disclosure also provide an operating method of the in vivo duct repair device of embodiments of the present disclosure, the method including: inserting the introducing end 20A of the catheter 20 into the to-be-repaired area 91 in the in vivo duct 90; extending the nozzle assembly into the catheter 20 and extending the spraying end 10A of the nozzle assembly out of the introducing end 20A of the catheter 20; and driving, by the drive device 40, the nozzle assembly 10 to spray the first material and the second material into the to-be-repaired area 91.

The operating method of the in vivo duct repair device of embodiments of the present disclosure has same advantages as the in vivo duct repair device of embodiments of the present disclosure.

The in vivo duct repair device and the operating method thereof in embodiments of the present disclosure are described below in conjunction with Figs. 1 to 35.

Fig. 1 is a schematic structural diagram of an in vivo duct repair device of an embodiment of the present disclosure. As shown in Fig. 1, the in vivo duct repair device includes a catheter 20, a nozzle assembly 10, a drive device 40 and a communication pipe 30.

The nozzle assembly 10 may be, for example, a nozzle assembly 100, a nozzle assembly 200, a nozzle assembly 300 and a nozzle assembly 400 in the following embodiments. For corresponding structures described in the nozzle assembly 10 in the following description, reference may be made to corresponding structures of the nozzle assemblies 100, 200, 300, 400 in the following embodiments and their reference signs.

The catheter 20 includes an introducing end 20A configured to be inserted into an in vivo duct 90. Two axial ends of the nozzle assembly 10 include a supplying end 10B and a spraying end 10A, respectively. The nozzle assembly 10 has a first material flow channel P1, a first material spout J1, a second material flow channel P2 and a second material spout J2. The first material flow channel P1 is arranged inside the nozzle assembly. The first material spout J1 is communicated with the first material flow channel P1 and is arranged on a side face of the spraying end 10A to spray a first material radially outwards of the nozzle assembly 10. The second material flow channel P2 is arranged inside the nozzle assembly 10 and is isolated from the first material flow channel P1; and the second material spout J2 is communicated with the second material flow channel P2 and is arranged on a side face of the spraying end 10A to spray a second material radially outwards of the nozzle assembly 10. The nozzle assembly 10 is configured to enter the catheter 20 and extend the spraying end 10A out of the introducing end 20A of the catheter 20 to spray the first material and the second material to an inner wall of the in vivo duct 90. The second material spout J2 is arranged staggeredly with respect to the first material spout J1 in an axial direction of the nozzle assembly 10.

The drive device 40 is configured to be connected to the supplying end 10B of the nozzle assembly and is configured to drive the nozzle assembly to spray the second material and the first material. The drive device 40 includes a first material supply part 41 and a second material supply part 42. The first material supply part 41 is in communication with the first material flow channel P1 to supply the first material to the first material flow channel P1. The second material supply part 42 is in communication with the second material flow channel P2 to supply the second material to the second material flow channel P2. The first material supply part 41 is a first injection device storing the first material, and the second material supply part is a second injection device storing the second material.

The communication pipe 30 includes a first tube 31 and a second tube 32, the first tube 31 being connected between the first injection device and the first material flow channel P1, and the second tube 32 being connected between the second injection device and the second material flow channel P2.

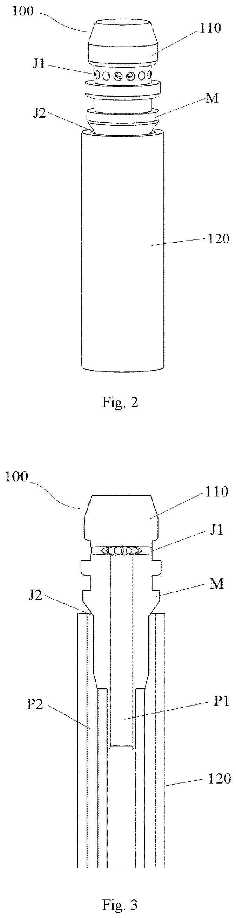

The nozzle assembly of the in vivo duct repair device of some embodiments of the present disclosure will be described in detail below in conjunction with Figs. 2 to 24.

Figs. 2 to 5 show a nozzle assembly 100 of an embodiment of the present disclosure. As shown in Figs. 2 to 5, the nozzle assembly 100 has a spraying end that sprays repair materials, and has a first material flow channel P1, first material spouts J1, a second material flow channel P2 and a second material spout J2. The first material flow channel P1 is arranged inside the nozzle assembly 100. The first material spouts J1 are communicated with the first material flow channel P1 and are located on a side face of the spraying end. The second material flow channel P2 is arranged inside the nozzle assembly 100 and staggered is isolated from the first material flow channel P1. The second material spout J2 is communicated with the second material flow channel P2 and is located on a side face of the spraying end, and the second material spout J2 is arranged staggeredly with respect to the first material spout J1 in an axial direction of the nozzle assembly 100.

The first material spout J1 is closer to an end face of the spraying end in the axial direction of the nozzle assembly 100 than the second material spout J2. The plurality of first material spouts J1 are uniformly distributed circumferentially of the nozzle assembly 100. The second material spout J2 is an annular spout.

As shown in Figs. 2 and 3, the nozzle assembly 100 further includes a medical adhesive scrape coating part M arranged on a side face of the nozzle assembly 100, the medical adhesive scrape coating part M being located between the first material spouts J1 and the second material spout J2 in the axial direction of the nozzle assembly 100. The medical adhesive scrape coating part M includes an annular projection arranged on a side face of the nozzle assembly. To facilitate coating, a side of the annular projection close to the second material spout J2 is inclined toward the side of the first material spouts J1 from a radially inner side to a radially outer side of the nozzle assembly 100.

As shown in Figs. 2 to 5, the nozzle assembly 100 includes a first nozzle body 110 and a second nozzle body 120. The first material spouts J1 are arranged on the first nozzle body 110. The second nozzle body 120 is arranged at the outer periphery of the first nozzle body 110. The second material spout J2 is arranged between the first nozzle body 110 and the second nozzle body 120. As shown in Fig. 3, in this embodiment, the second material spout J2 is arranged between a circumferential outer wall of the first nozzle body 110 and an inner wall of an end of the second nozzle body 120 close to the spraying end.

The first material flow channel P1 is arranged in the first nozzle body 110. The plurality of first material spouts J1 are communicated with the first material flow channel P1.

The second material flow channel P2 is arranged between the second nozzle body 120 and the first nozzle body 110. The second material flow channel P2 is generally an annular flow channel arranged at the outer periphery of the first material flow channel P1, and the second material flows axially of the annular flow channel. An outlet of the second material flow channel P2 (second material discharge port) is the second material spout J2.

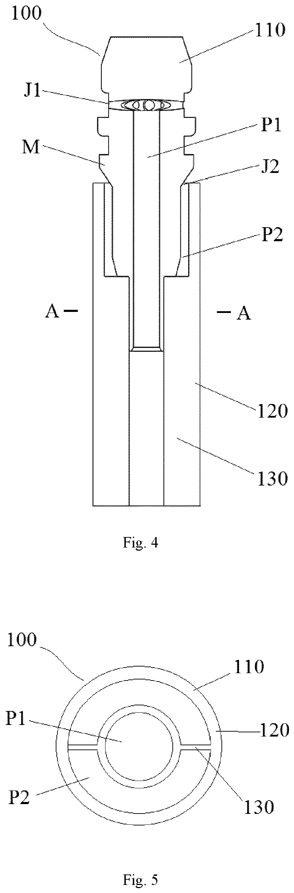

As shown in Figs. 4 and 5, the nozzle assembly 100 further includes a connecting piece 130, the connecting piece 130 being connected between the first nozzle body 110 and the second nozzle body 120.

The nozzle assembly 100 of the embodiment of the present disclosure is described in more detail below in conjunction with Figs. 2 to 5.

As shown in Figs. 2 to 5, the nozzle assembly 100 includes a first nozzle body 110 and a second nozzle body 120 that are arranged coaxially. The first nozzle body 110 is inserted into the second nozzle body 120. The first nozzle body 110 and the second nozzle body 120 are fixed to each other by a connecting piece 130. The connecting piece 130 is used to fix the inner-layer first nozzle body 110 and the outer-layer second nozzle body 120, thereby maintaining the coaxiality of the first nozzle body 110 and the second nozzle body 120.

In this embodiment, an upper end of the first nozzle body 110 is a spraying end of the nozzle assembly 100, and the upper end of the first nozzle body 110 is provided with a number of first material spouts J1, the plurality of first material spouts J1 being arranged uniformly on a side wall of the upper end of the first nozzle body 110 circumferentially of the nozzle assembly 100, so that the first material can be sprayed from the first material spouts J1 respectively. Since the first material spouts J1 are uniformly distributed on the side wall of the upper end of the first nozzle body 110, the first material can be changed into several streams of fluid and sprayed uniformly.

A first material flow channel P1 is arranged in the first nozzle body 110, and the first material flow channel P1 has a first material discharge port at one end (an upper end in the figures) and a first material feed port at the other end (a lower end in the figures). The first material discharge port is communicated with the first material spouts J1. An annular space between an inner wall of the second nozzle body 120 and an outer wall of the first nozzle body 110 forms a second material flow channel P2, and the second material flow channel P2 has a second material discharge port, i.e. a second material spout J2, at one end (an upper end in the figures), and a second material feed port at the other end (a lower end in the figures).

To make the medical adhesive sprayed on an inner wall of the in vivo duct 90 more flat, a medical adhesive scrape coating part M is provided at a position on the first nozzle body 110 close to the medical adhesive spout J2. In Figs. 2 and 3, the medical adhesive scrape coating part M is in the shape of an inverted cone.

The nozzle assembly 100 of the embodiment of the present disclosure facilitates the coating of the medical adhesive and the bio-ink on the inner wall of the in vivo duct, such as the inner wall of long and narrow lumen tissues. A feasible working process of the nozzle assembly 100 is described below with the first material spout J1 spraying the bio-ink and the second material spout J2 spraying the medical adhesive as an example.

To start coating, the nozzle assembly 100 is extended into the first end 91A of the to-be-repaired area 91 of the in vivo duct 90 away from the catheter 20 along the catheter 20 inserted into the in vivo duct 90, and the position of the second material spout J2 is aligned with the first end 91A of the to-be-repair area 91, at the same time the drive device 40 starts to drive the second material spout J2 of the nozzle assembly 100 to spray the medical adhesive, and causes the nozzle assembly 100 to move towards the second end 91B of the to-be-repaired area 91; in the process of movement, as the positions of the first material spout J1 and the second material spout J2 are staggered, the second material sprayed out can be first coated to the inner wall of the in vivo duct 90 to form a medical adhesive layer 91D; as the nozzle assembly 100 moves relative to the in vivo duct 90, the drive device 40 starts to drive the first material spout J1 of the nozzle assembly 100 to spray the bio-ink, which is coated on the medical adhesive layer 91D, and at the same time, due to the adhering effect of the medical adhesive, the bio-ink can be immediately adhered to the medical adhesive layer 91D; and when the nozzle assembly 100 completely exits the in vivo duct 90, the medical adhesive layer 91D and a bio-ink layer 91C can be formed on the inner wall of the in vivo duct 90 at the same time, thus completing the repair of the to-be-repaired area 91 of the in vivo duct 90.

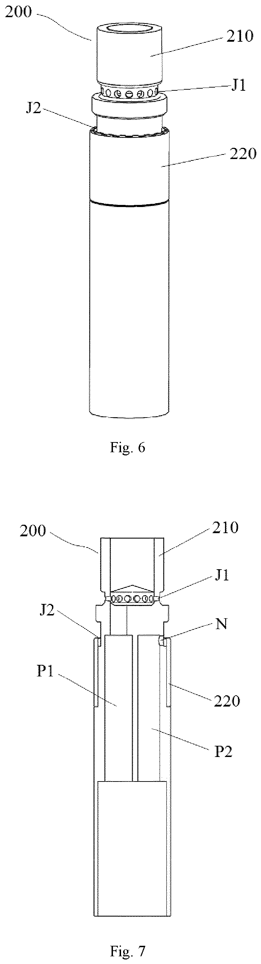

Figs. 6 and 7 show a nozzle assembly 200 of an embodiment of the present disclosure. As shown in Figs. 6 and 7, the nozzle assembly 200 has a spraying end that sprays repair materials, and has a first material flow channel P1, first material spouts J1, a second material flow channel P2 and a second material spout J2. The first material flow channel P1 is arranged inside the nozzle assembly 200. The first material spouts J1 are communicated with the first material flow channel P1 and are located on a side face of the spraying end. The second material flow channel P2 is arranged inside the nozzle assembly 200 and is isolated from the first material flow channel P1. The second material spout J2 is communicated with the second material flow channel P2 and is located on a side face of the spraying end, and the second material spout J2 is arranged staggeredly with respect to the first material spouts J1 in an axial direction of the nozzle assembly 200.

The first material spouts J1 are closer to an end face of the spraying end in the axial direction of the nozzle assembly 200 than the second material spout J2. The plurality of first material spouts J1 are uniformly distributed circumferentially of the nozzle assembly 200. The second material spout J2 is an annular spout.

As shown in Figs. 6 and 7, the nozzle assembly 200 includes a first nozzle body 210 and a second nozzle body 220. The first material spouts J1 are arranged on the first nozzle body 210. The second nozzle body 220 is arranged at the outer periphery of the first nozzle body 210.

The first material flow channel P1 is arranged in the first nozzle body 210. The plurality of first material spouts J1 are communicated with the first material flow channel P1.

The second material flow channel P2 is arranged in the first nozzle body 210, and a side wall of the first nozzle body 210 has a communication port N communicated with the second material flow channel P2.

The second material spout J2 is arranged between the first nozzle body 210 and the second nozzle body 220. The second material spout J2 includes an annular spout arranged around the axial direction of the nozzle assembly, formed by the first nozzle body 210 and an end of the second nozzle body 220 close to the spraying end, the communication port N being communicated with the annular spout. As shown in Fig. 7, in this embodiment, the second material spout J2 is arranged between a circumferential outer wall of the first nozzle body 210 and an inner wall of the end of the second nozzle body 220 close to the spraying end.

The nozzle assembly 200 of the embodiment of the present disclosure is described in more detail below in conjunction with Figs. 6 and 7.

As shown in Figs. 6 and 7, the nozzle assembly 200 includes a first nozzle body 210 and a second nozzle body 220. A first material flow channel P1 and a second material flow channel P2 are arranged in parallel in the first nozzle body 210, the first material flow channel P1 having a first material discharge port at one end (an upper end in the figures) and a first material feed port at the other end (a lower end in the figures), and the second material flow channel P2 having a second material discharge port at one end (an upper end in the figures) and a second material feed port at the other end (a lower end in the figures). An upper end of the first nozzle body 210 is provided with a plurality of first material spouts J1, the plurality of first material spouts J1 being arranged uniformly on a side wall of the upper end of the first nozzle body 210 circumferentially of the nozzle assembly 200, and the first material spouts J1 being communicated with the first material discharge port. A central part of the first nozzle body 210 is provided with a boss, and the sleeve-like second nozzle body 220 is sleeved on the boss. An annular spout between the second nozzle body 220 and the first nozzle body 210 is a second material spout J2. The second material spout J2 is communicated with the second material discharge port of the second material flow channel P2.

The nozzle assembly 200 achieves the same technical effect that when the medical adhesive is first sprayed on the inner wall of the in vivo duct, and at the same time the bio-ink is sprayed on the medical adhesive layer in a relatively short period of time, a double-layer structure with the medical adhesive layer and the bio-ink layer is formed on the inner wall of the in vivo duct by moving once (that is, the nozzle assembly is gradually moved from the farthest position from the introducing end 20A to the introducing end 20A), as the technical effect achieved by the nozzle assembly 100, and repeated description is omitted here.

The diameters of the first material flow channel P1 and the second material flow channel P2 in the nozzle assembly 200 can be set smaller than the diameters of the corresponding flow channels in the nozzle assembly 100, so that the amounts of the first material and the second material can be reduced in the coating process.

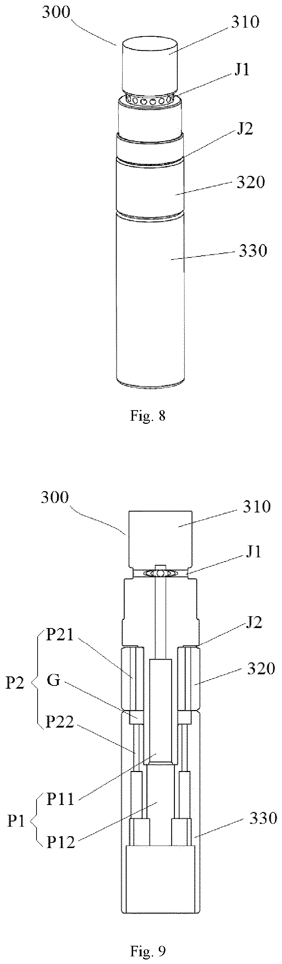

Figs. 8 to 16 show a nozzle assembly 300 of an embodiment of the present disclosure. As shown in Figs. 8 to 16, the nozzle assembly 300 has a spraying end (an upper end in the figures) that sprays repair materials, and has a first material flow channel P1, first material spouts J1, a second material flow channel P2 and a second material spout J2.

The first material flow channel P1 is arranged inside the nozzle assembly 300. The first material spouts J1 are communicated with the first material flow channel P1 and are located on a side face of the spraying end. The second material flow channel P2 is arranged inside the nozzle assembly 300 and is isolated from the first material flow channel P1. The second material spout J2 is communicated with the second material flow channel P2 and is located on a side face of the spraying end, and the second material spout J2 is arranged staggeredly with respect to the first material spouts J1 in an axial direction of the nozzle assembly 300.

The first material spouts J1 are closer to an end face of the spraying end in the axial direction of the nozzle assembly 300 than the second material spout J2. The plurality of first material spouts J1 are uniformly distributed circumferentially of the nozzle assembly 300. The second material spout J2 is an annular spout.

As shown in Figs. 8 to 16, the nozzle assembly 300 includes a first nozzle body 310, a second nozzle body 320 and a nozzle mounting seat 330. The first nozzle body 310 and the second nozzle body 320 are arranged on the nozzle mounting seat 330.

The first material spouts J1 are arranged on the first nozzle body 310. The second nozzle body 320 is of a sleeve-like structure and is arranged at the outer periphery of the first nozzle body 310.

The second material spout J2 includes an annular spout formed by the first nozzle body 310 and an end of the second nozzle body 320 close to the spraying end, a communication port being communicated with the annular spout. As shown in Fig. 9, the second material spout J2 is arranged between a bottom wall of a middle protrusion of the first nozzle body 310 and an end wall of the second nozzle body 320 close to the spraying end of the nozzle assembly 300.

The first material flow channel P1 includes a first material nozzle flow channel P11 arranged in the first nozzle body 310 and a first material mounting seat flow channel P12 arranged in the nozzle mounting seat 300.

The second material flow channel P2 includes a second material nozzle flow channel P21 arranged in the second nozzle body 320 and a second material mounting seat flow channel P22 arranged in the nozzle mounting seat. As shown in Fig. 9, the second material flow channel P2 further includes a first annular buffer groove G, the second material nozzle flow channel P21 and the second material mounting seat flow channel P22 being communicated through the first annular buffer groove G. As shown in Fig. 9, the first annular buffer groove G is formed between the second nozzle body 320 and the nozzle mounting seat 330.

The nozzle assembly 300 of the embodiment of the present disclosure is further described below in conjunction with Figs. 8 to 16.

As shown in Figs. 8 to 16, the nozzle assembly 300 includes a first nozzle body 310, a second nozzle body 320 and a nozzle mounting seat 330. The second nozzle body 320 and the first nozzle body 310 are successively sleeved on the nozzle mounting seat 330, and the first nozzle body 310 passes through the second nozzle body 320 and is inserted into the nozzle mounting seat 330.

A first material mounting seat flow channel P12 is arranged in the nozzle mounting seat 330, and the first material mounting seat flow channel P12 has a feed port of the first material mounting seat flow channel P12 at one end and a discharge port of the first material mounting seat flow channel P12 at the other end. Second material mounting seat flow channels P22 are arranged symmetrically on two sides of the first material mounting seat flow channel P12. In embodiments not shown, one second material mounting seat flow channel P22 may be provided in order to reduce the amount of the second material. The second material mounting seat flow channel P22 has a discharge port of the second material mounting seat flow channel P22 at one end and a feed port of the second material mounting seat flow channel P22 at the other end.

The first nozzle body 310 includes a mounting post 311 arranged in the lower part thereof, and the second nozzle body 320 is provided with a mounting cavity C2 in the middle, the mounting cavity C2 being adapted for the mounting post 311 of the first nozzle body 310 to pass therethrough. A plurality of second material nozzle flow channels P21 are uniformly arranged at the outer side of the mounting cavity C2. Each second material nozzle flow channel P21 has a second material first discharge port O2 at one end and a second material first feed port at the other end. An upper end surface of the second nozzle body 320 is provided with a number of second material first discharge ports O2, and the number of second material first discharge ports O2 are uniformly arranged circumferentially. Moreover, the second material first discharge ports O2 are communicated with the second material spouts J2, and the second material first feed port is communicated with a second material first discharge port.

As shown in Fig. 9, the second material mounting seat flow channels P22 are arranged symmetrically on two sides of the first material mounting seat flow channel P12, and a first annular buffer groove G is arranged above the second material mounting seat flow channels P22, and the second material mounting seat flow channel P22 has a second material first discharge port at one end and a second material second feed port at the other end. The second material second discharge port is communicated with the first annular buffer groove G. The mounting cavity C2 is arranged in the middle of the second nozzle body 320, and a number of second material nozzle flow channels P21 are uniformly arranged at the outer periphery of the mounting cavity C2, each second material nozzle flow channel P21 having at one end the second material first discharge port O2 communicated with the second material spout, and at the other end the second material first feed port communicated with the first annular buffer groove G.

An upper end of the first nozzle body 310 is provided with a number of first material spouts J1, the number of first material spouts J1 being arranged uniformly on a side wall of the upper end of the first nozzle body 310. The mounting post 311 is arranged at the lower end of the first nozzle body 310, and a first material nozzle flow channel P11 is arranged in the first nozzle body 310, the first material nozzle flow channel P 11 having a first material first discharge port at one end and a first material first feed port at the other end, the first material first discharge port being communicated with the first material spouts J1, and the first material first feed port being communicated with the first material second discharge port.

The nozzle assembly 300 achieves the same technical effect that when the medical adhesive is first sprayed on the inner wall of the in vivo duct, and at the same time the bio-ink is sprayed on the medical adhesive layer in a relatively short period of time, a double-layer structure with the medical adhesive layer and the bio-ink layer is formed on the inner wall of the in vivo duct by moving once (that is, the nozzle assembly is gradually moved from the farthest position from the introducing end 20A to the introducing end 20A), as the technical effect of the nozzle assembly 100, and repeated description is omitted here.

The diameters of the first material flow channel and the second material flow channel in the nozzle assembly 300 become smaller than the diameters of the corresponding flow channels in the nozzle assembly 100, so that the amounts of the first material and the second material are reduced in the coating process.

In the nozzle assembly 300, the upper end of the nozzle mounting seat 330 and the lower end of the mounting post 311 may be of a threaded connection structure to facilitate assembly and disassembly of the nozzle mounting seat 330, the first nozzle body 310 and the second nozzle body 320.

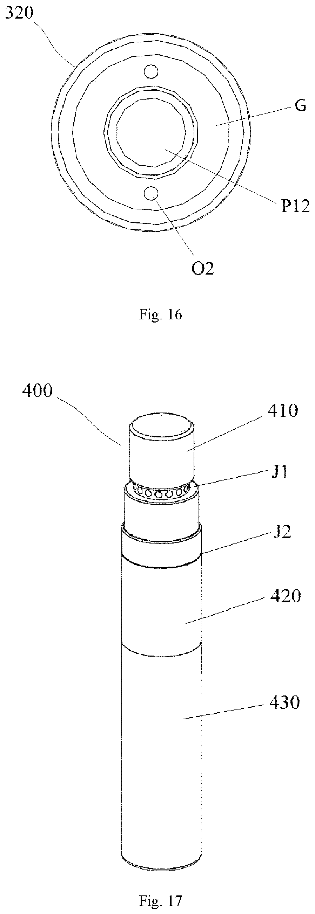

Figs. 17 to 24 show a nozzle assembly 400 of an embodiment of the present disclosure. As shown in Figs. 17 to 24, the nozzle assembly 400 has a spraying end (an upper end in the figures) that sprays repair materials, and has a first material flow channel P1, first material spouts J1, a second material flow channel P2 and a second material spout J2.

The first material flow channel P1 is arranged inside the nozzle assembly 400. The first material spouts J1 are communicated with the first material flow channel P1 and are located on a side face of the spraying end. The second material flow channel P2 is arranged inside the nozzle assembly 400 and is isolated from the first material flow channel P1. The second material spout J2 is communicated with the second material flow channel P2 and is located on a side face of the spraying end, and the second material spout J2 is arranged staggeredly with respect to the first material spouts J1 in an axial direction of the nozzle assembly 400.

The first material spouts J1 are closer to an end face of the spraying end in the axial direction of the nozzle assembly 400 than the second material spout J2. The plurality of first material spouts J1 are uniformly distributed circumferentially of the nozzle assembly 400, and the second material spout J2 is an annular spout.

As shown in Figs. 7 to 24, the nozzle assembly includes a first nozzle body 410, a second nozzle body 420 and a nozzle mounting seat 430. The first nozzle body 410 and the second nozzle body 420 are arranged on the nozzle mounting seat 430.

The first material spouts J1 are arranged on the first nozzle body 410. The second nozzle body 420 is of a sleeve-like structure and is arranged at the outer periphery of the first nozzle body 410.

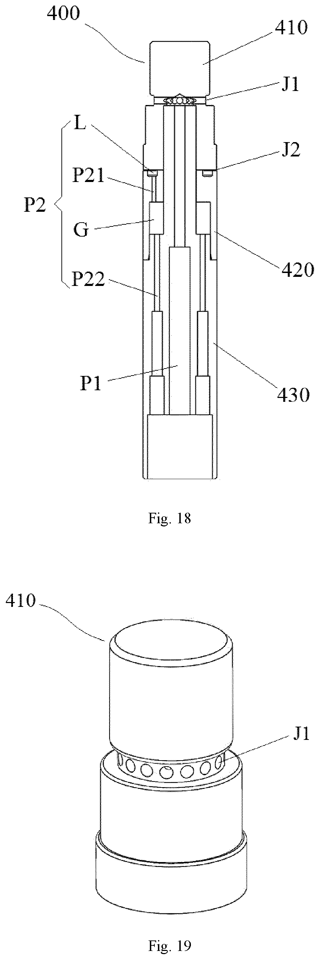

The second material spout J2 includes an annular spout formed by the first nozzle body 410 and an end of the second nozzle body 420 close to the spraying end. As shown in Fig. 18, the second material spout J2 is arranged between a bottom wall of a middle protrusion of the first nozzle body 410 and an end face of the second nozzle body 420 close to the spraying end of the nozzle assembly 400.

The first material flow channel P1 is arranged inside the nozzle mounting seat 430.

The second material flow channel P2 includes a second material nozzle flow channel P21 arranged in the second nozzle body 420 and a second material mounting seat flow channel P22 arranged in the nozzle mounting seat 430. As shown in Fig. 18, the second material flow channel P2 further includes a first annular buffer groove G, the second material nozzle flow channel P21 and the second material mounting seat flow channel P22 being communicated through the first annular buffer groove G. As shown in Fig. 18, the first annular buffer groove G is formed between the second nozzle body 420 and the nozzle mounting seat 430.

As shown in Fig. 18, the second material flow channel P2 includes a second annular buffer groove L arranged between the second material spout J2 and the second material nozzle flow channel P21.

The nozzle assembly 400 of the embodiment of the present disclosure is further described below in conjunction with Figs. 17 to 24.

As shown in Figs. 17 to 24, the nozzle assembly 400 includes a first nozzle body 410, a second nozzle body 420 and a nozzle mounting seat 430. The second nozzle body 420 and the first nozzle body 410 are successively sleeved on the nozzle mounting seat 430.

A first material flow channel P1 is arranged in the nozzle mounting seat 430, and the first material flow channel P1 has a first material discharge port O1 at one end (an upper end in the figures) and a first material feed port at the other end (a lower end in the figures).

Second material mounting seat flow channels P22 are arranged symmetrically on two sides of the first material flow channel P1 in the nozzle mounting seat 430, respectively. In some embodiments not shown, each second material mounting seat flow channel P22 has a second material second discharge port O22 at one end and a second material second feed port at the other end. One second material mounting seat flow channel P22 may be provided in order to reduce the amount of the second material.

The nozzle mounting seat 430 is provided from top to bottom with a first mounting post 431 for mounting the first nozzle body 410, a second mounting post 432 for mounting the second nozzle body 420, and a mounting seat body 433, respectively. An upper outer wall of the first mounting post 431 is provided with a first external thread, and an outer wall of the second mounting post 432 is provided with a second external thread. The second material second discharge port O22 is located on an upper end face of the second mounting post 432. The first material discharge port O1 is located at an upper end face of the first mounting post 431.

Inside the second nozzle body 420 are successively arranged a first mounting cavity C21 adapted for the first mounting post 431 to pass therethrough and a second mounting cavity C22 adapted for the second mounting post 432 to pass therethrough. An inner wall of the second mounting cavity C22 is provided with a second internal thread T2, and a detachable connection between the second nozzle body 420 and the nozzle mounting seat 430 is achieved by a threaded fit between the second internal thread T2 and the second external thread.

An upper end surface of the second nozzle body 420 is provided with a second annular buffer groove L. A bottom face of the second annular buffer groove L is provided with a number of second material first discharge ports 021, the number of second material first discharge ports 021 being uniformly distributed on the bottom face of the second annular buffer groove L. A number of second material nozzle flow channels P21 are provided below the second annular buffer groove L correspondingly to the number of second material discharge ports 021. Each second material nozzle flow channel P21 has a second material first discharge port 021 at one end (an upper end in the figures) and a second material first feed port at the other end (a lower end in the figures). The second material first discharge port 021 is communicated with the second material spout J2. The second material first feed port is communicated with the second material second discharge port O22.

Inside the first nozzle body 410 is arranged a mounting cavity C1 adapted for cooperative installation with the first mounting post 431. An inner wall of the mounting cavity C1 is provided with a first internal thread T1. A detachable connection between the first nozzle body 410 and the nozzle mounting seat 430 is achieved by a threaded fit between the first internal thread T1 and the first external thread.

By detachably connecting the second nozzle body 420 and the first nozzle body 410 respectively to the nozzle mounting seat 430, the second nozzle body 420 and the first nozzle body 410 can be conveniently removed, mounted and cleaned.

An upper end of the first nozzle body 410 is provided with a number of first material spouts J1, the number of first material spouts J1 being circumferentially arranged uniformly on a side wall of the upper end of the first nozzle body 410, and the first material spouts J1 being communicated with the first material discharge port O1.

To ensure that the second material sprayed from the second material spout J2 is coated on the inner wall of the in vivo duct 90 more uniformly, a sponge brush is provided at a gap between a lower end of the first nozzle body 410 and an upper end of the second nozzle body 420. The second material sprayed from the second material spout J2 can first moisten the sponge brush such that the sponge brush is wetted, and then the sponge brush is brought into contact with and squeezed against the inner wall of the in vivo duct 90, thereby uniformly coating the second material on the inner wall of the in vivo duct 90.

The nozzle assembly 400 achieves the same technical effect that when the medical adhesive is first sprayed on the inner wall of the in vivo duct, and at the same time the bio-ink is sprayed on the medical adhesive layer in a relatively short period of time, a double-layer structure with the medical adhesive layer and the bio-ink layer is formed on the inner wall of the in vivo duct by moving once (that is, the nozzle assembly is gradually moved from the farthest position from the introducing end 20A to the introducing end 20A), as the technical effect of the nozzle assembly 100, and repeated description is omitted here.

The diameters of the first material flow channel P1 and the second material flow channel P2 in the nozzle assembly 400 become smaller than the diameters of the corresponding flow channels in the nozzle assembly 100, so that the amounts of the first material and the second material can be reduced in the coating process. By changing the structure of the second material spout J2 in the second nozzle body 420 and adding the sponge brush, the second material can be coated on the inner wall of the in vivo duct 90 more uniformly in the coating process.

An operating process of the in vivo duct repair device of the present disclosure is described below in conjunction with Figs. 25 to 29.

Fig. 25 is a structural diagram of the in vivo duct 90 to be repaired. The in vivo duct 90 is, for example, a human blood vessel. Two ends of the to-be-repaired area 91 in the in vivo duct 90 in an extending direction of the in vivo duct 90 are the first end 91A and the second end 91B, respectively.

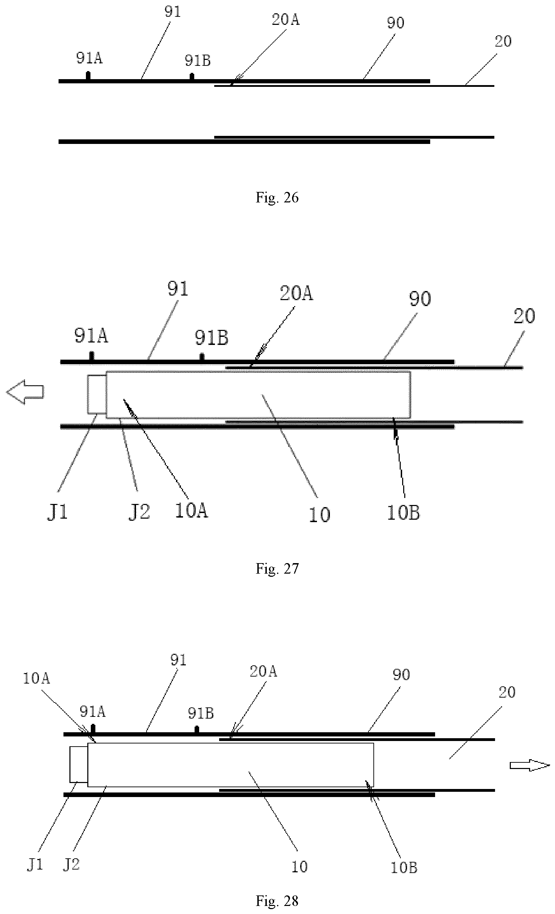

Fig. 26 is a schematic structural diagram illustrating the introducing end 20A of the catheter 20 is inserted into the in vivo duct 90. Fig. 27 is a schematic structural diagram illustrating the spraying end 10A of the nozzle assembly is extended from the introducing end 20A of the catheter 20 to an initial repair position. Fig. 28 is a schematic structural diagram illustrating the spraying end 10A of the nozzle assembly starts coating. The communication pipe 30 and the drive device 40 are not shown in Figs. 26 to 28.

An operating process of repairing the in vivo duct 90 using the in vivo duct repair device of the above embodiments of the present disclosure is as follows with the first material spout J1 spraying the bio-ink and the second material spout J2 spraying the medical adhesive as an example:

The catheter 20 is delivered to a first target position in the in vivo duct 90. The first target position may be near the second end 91B of the to-be-repaired area 91 of the in vivo duct 90 shown in Figs. 25 to 29 so that the catheter 20 forms a passage for the nozzle assembly 10 to reach the target position in the in vivo duct 90. Then the nozzle assembly 10 is introduced into the catheter 20 and delivered through the catheter 20 to a second target position in the in vivo duct 90. The second target position may be near the first end 91A of the to-be-repaired area 91 of the in vivo duct 90 shown in Figs. 25 to 29 where the second material spout J2 of the nozzle assembly 10 is delivered. The delivery process of the catheter 20 and nozzle assembly 10 can be assisted by ultrasound imaging technology, thereby achieving precise positioning during delivery.

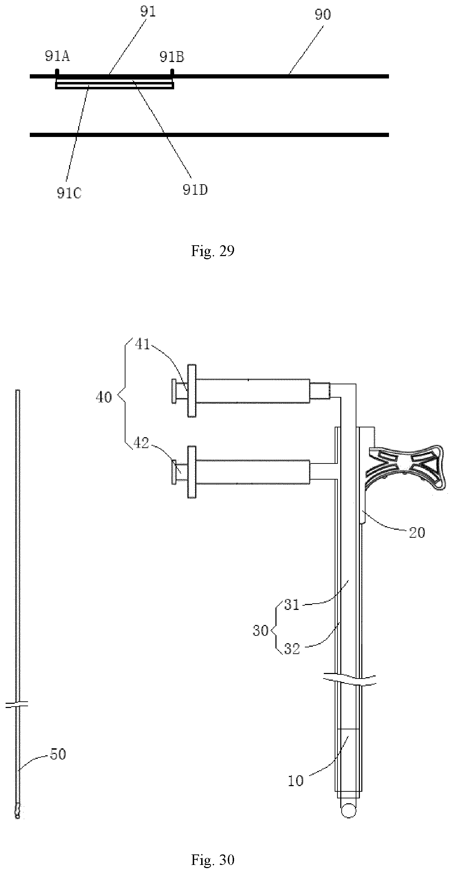

The bio-ink and the medical adhesive are supplied to the nozzle assembly 10 by the drive device 40, so that the bio-ink can be sprayed from the first material spout J1 and the medical adhesive can be sprayed from the second material spout J2. When coating begins, the drive device 40 supplies the bio-ink and the medical adhesive to the nozzle assembly 10, and since the axial positions of the first material spout J1 and the second material spout J2 are staggered, the medical adhesive, when being sprayed, comes into contact with the inner wall of the in vivo duct 90 before the bio-ink, and as the nozzle assembly 10 moves toward the second end 91B of the to-be-repaired area 91 (i.e. moving in a direction indicated by an arrow in Fig. 28), the medical adhesive is coated on the inner wall of the in vivo duct 90, and the medical adhesive layer 91D can be formed on the inner wall of the in vivo duct 90 due to the adhering effect of the medical adhesive; with the movement of the nozzle assembly 10, the bio-ink comes into contact with the medical adhesive layer 91D at short time after the medical adhesive comes into contact with the inner wall of the in vivo duct 90, and the bio-ink can be adhered to the medical adhesive layer 91D due to the adhering effect of the medical adhesive; and with the continuous movement of the nozzle assembly 10, the bio-ink is coated on the medical adhesive layer 91D, to form the bio-ink layer 91C on the medical adhesive layer 91D, thus ensuring the formation of the medical adhesive layer 91D and the bio-ink layer 91C on the inner wall of the in vivo duct 90, thereby achieving repair of a target area (e.g. a damaged site) in the in vivo duct 90 of a patient. Fig. 29 is a structural diagram of the in vivo duct 90 that has been repaired.

Fig. 30 is a schematic structural diagram of an in vivo duct repair device of an embodiment of the present disclosure. Compared with the in vivo duct repair devices of the embodiments shown in Figs. 1 to 29, this embodiment is added with a guide wire 50. The guide wire 50 is configured to form an initial pathway within the in vivo duct 90. The catheter 20 is inserted into the in vivo duct 90 along the guide wire 50.

In conjunction with Figs. 25 to 29, a repair process of repairing the in vivo duct 90 using the in vivo duct repair device of this embodiment is described as follows.

First, the guide wire 50 is introduced into the in vivo duct 90 to obtain the initial passageway within the in vivo duct 90, and then the catheter 20 is delivered to the first target position in the in vivo duct 90 by introducing the catheter 20 along the guide wire 50. The first target position may be near the second end 91B of the to-be-repaired area 91 of the in vivo duct 90 shown in Figs. 25 to 29 so that the catheter 20 forms a passage for the nozzle assembly 10 to reach the target position in the in vivo duct 90. Then the guide wire 50 is drawn out, and the nozzle assembly is introduced into the catheter 20 and delivered through the catheter 20 to the second target position in the in vivo duct 90. The second target position may be near the first end 91A of the to-be-repaired area 91 of the in vivo duct 90 shown in Figs. 25 to 29 where the second material spout J2 of the nozzle assembly 10 is delivered. The delivery process can be assisted by ultrasound imaging technology, thereby achieving precise positioning during delivery.

When coating begins, the drive device 40 first drives the nozzle assembly 100 to spray the medical adhesive so that the medical adhesive can be sprayed from the wall of second material spout J2, and at that time the nozzle assembly 10 is caused to move toward the second end 91B of the to-be-repaired area 91; in the moving process of the nozzle assembly 10, the sprayed medical adhesive comes into contact with the inner wall of the in vivo duct 90, and with the continuous movement of the nozzle assembly 10, the medical adhesive is coated on the inner wall of the in vivo duct 90, and the medical adhesive layer 91D can be formed on the inner wall of the in vivo duct 90 due to the adhering effect of the medical adhesive; when the first material spout J1 moves to the position of the first end 91A of the to-be-repaired area 91, the drive device 40 drives the nozzle assembly 10 to spray the bio-ink so that the bio-ink can be sprayed from the first material spout J1, and at that time the nozzle assembly 10 is caused to move further toward the second end 91B of the to-be-repaired area 91; in the moving process of the nozzle assembly 10, the sprayed bio-ink comes into contact with the medical adhesive layer 91D, and at the same time the bio-ink can be adhered to the medical adhesive layer 91D due to the adhering effect of the medical adhesive; and with the continuous movement of the nozzle assembly 10, the bio-ink is coated on the medical adhesive layer 91D, to form the bio-ink layer 91C on the medical adhesive layer 91D, thus ensuring the formation of the medical adhesive layer 91D and the bio-ink layer 91C on the inner wall of the in vivo duct 90, thereby achieving repair of a target area (e.g. a damaged site) in the in vivo duct 90 of a patient.

To adapt to different in vivo ducts 90, the catheter 20, the guide wire and the nozzle assembly can be set to sizes adapted to the in vivo ducts 90.

For other parts in this embodiment that are not described, reference may be made to relevant parts of the embodiments described above.

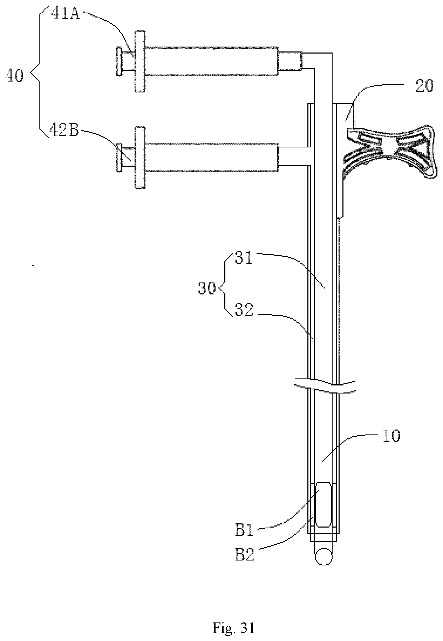

Fig. 31 is a schematic structural diagram of an in vivo duct repair device of an embodiment of the present disclosure. This in vivo duct repair device differs from the in vivo duct repair devices in the above embodiments in that: the nozzle assembly includes a first material storage part, and the drive device 40 includes a first drive part 41A, an outlet of the first material storage part being communicated with the first material flow channel P1, the first drive part 41A being coupled with the first material storage part to drive the first material storage part to output the first material from the outlet thereof. The nozzle assembly includes a second material storage part, and the drive device 40 includes a second drive part 41B, an outlet of the second material storage part being communicated with the second material flow channel P2, the second drive part 41B being coupled with the second material storage part to drive the second material storage part to output the second material from the outlet thereof.

The first material storage part includes a first bag B1 storing the first material. The first drive part 41A includes a first fluid pump. The second material storage part includes a second bag B2 storing the second material, and the second drive part 41B includes a second fluid pump. The first fluid pump is a third injection device, and the second fluid pump is a fourth injection device.

Storing the coating materials by means of the storage bags facilitates release of the coating materials by fluid driving of the storage bags.

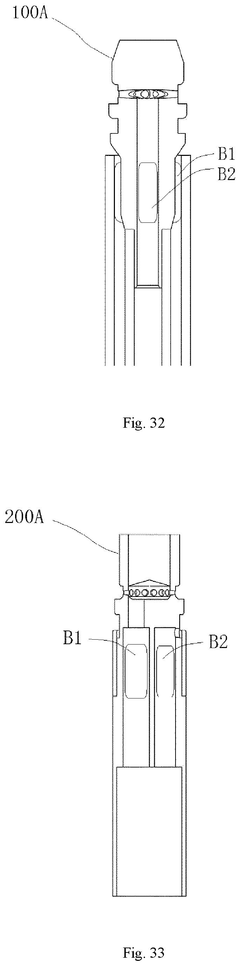

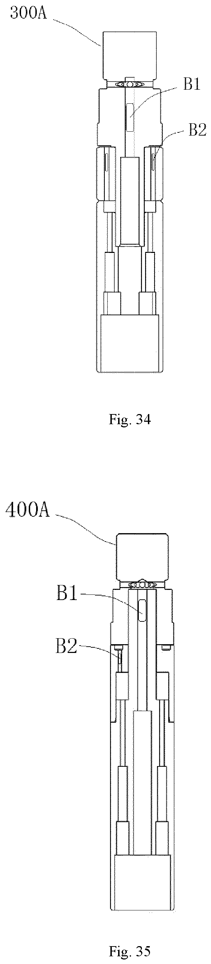

Fig. 32 is a three-dimensional structural diagram of a nozzle assembly 100A of an in vivo duct repair device of an embodiment of the present disclosure. Fig. 33 is a three-dimensional structural diagram of a nozzle assembly 200A of an in vivo duct repair device of an embodiment of the present disclosure. Fig. 34 is a three-dimensional structural diagram of a nozzle assembly 300A of an in vivo duct repair device of an embodiment of the present disclosure. Fig. 35 is a three-dimensional structural diagram of a nozzle assembly 400A of an in vivo duct repair device of an embodiment of the present disclosure.

Compared with the nozzle assembly 100, the nozzle assembly 200, the nozzle assembly 300 and the nozzle assembly 400 described above, the nozzle assembly 100A, the nozzle assembly 200A, the nozzle assembly 300A and the nozzle assembly 400A are added with a first bag B1 and a second bag B2 respectively. The nozzle assembly 100A, the nozzle assembly 200A, the nozzle assembly 300A and the nozzle assembly 400A can all be used as examples of the nozzle assembly 10 in the embodiment shown in Fig. 31.

In the nozzle assembly 100A, the nozzle assembly 200A, the nozzle assembly 300A and the nozzle assembly 400A, the first bag B1 is located in the first material flow channel P1 and separates the first material flow channel P1 into an upstream segment and a downstream segment, the first fluid pump being communicated with the upstream segment of the first material flow channel P1, an outlet of the first bag B1 being communicated with the downstream segment of the first material flow channel P1; and the second bag B2 is located in the second material flow channel P2 and separates the second material flow channel P2 into an upstream segment and a downstream segment, the second fluid pump being communicated with the upstream segment of the second material flow channel P2, and an outlet of the second bag B2 being communicated with the downstream segment of the second material flow channel P2.

The first bag B1 is made of a thin film material and is filled with the first material therein, and the second bag B1 is made of a thin film material and is filled with the second material therein; and the first bag B1 and the second bag B1 have mechanical properties that allow easy breaking by pressure. The first bag B1 is fixedly mounted, for example, at a discharge end of the first material flow channel P1, and has on a side facing the discharge end a first weak part that is easy to break relative to other parts of the first bag B1, and an opening formed after the first weak part is broken under pressure becomes the outlet of the first bag B1. The second bag B2 is fixedly mounted, for example, at a discharge end of the second material flow channel P2, and has on a side facing the discharge end a second weak part that is easy to break relative to other parts of the second bag B2, and an opening formed after the second weak part is broken under pressure becomes the outlet of the second bag B1.

For other parts of the nozzle assembly 100A, the nozzle assembly 200A, the nozzle assembly 300A and the nozzle assembly 400A that are not described, reference may be made correspondingly to relevant parts of the nozzle assembly 100, the nozzle assembly 200, the nozzle assembly 300 and the nozzle assembly 400 described above.

A process of repairing the in vivo duct 90 using the in vivo duct repair device of this embodiment differs from the repairing process in the above embodiment in that the first bag B1 is compressed by a fluid pumped by the first fluid pump so that the first material is released and sprayed from the first material spout J1, and the second bag B2 is compressed by a fluid pumped by the second fluid pump so that the second material is released and sprayed from the second material spout J2.

For other parts of the in vivo duct repair device and the operating process thereof that are not described, reference may be made to relevant parts of the in vivo duct repair devices of the above embodiments. In addition, Fig. 31 shows the in vivo duct repair device without a guide wire, while in an alternative embodiment of this embodiment, the device may be provided with a guide wire.

By providing the first bag B1 and the second bag B1 and fixedly mounting the same in the corresponding flow channels respectively, and pressing to open the first bag B1 and the second bag B1 by the fluid, such as gas, introduced into the flow channels, the supply of the repair material is implemented, and the distances for the repair materials to reach the corresponding spouts are shortened, thereby reducing the amounts of residues of the repair materials in the corresponding flow channels and being conducive to saving the repair materials.

To adapt to different in vivo ducts 90, the catheter 20, the guide wire 50 and the nozzle assembly 10 can be set to sizes adapted to the in vivo ducts 90.

Embodiments of the present disclosure can achieve double-layer coating on the inner wall of the in vivo duct 90, achieve the formation of the medical adhesive layer 91D and the bio-ink layer 91C at the same time on the damaged site of the in vivo duct 90, fit the bio-ink to the damaged site through the medical adhesive, and finally implement the repair of the damaged site by biological properties of the bio-ink.

In the above embodiments, the in vivo duct repair device and the operating process thereof in each of the embodiments of the present disclosure are described with the first material spout spraying the bio-ink and the second material spout spraying the medical adhesive as an example. Alternative embodiments of the in vivo duct repair devices in the above embodiments may also be constructed with the first material spout spraying the medical adhesive and the second material spout spraying the bio-ink. For the alternative embodiments, by controlling the timing and manner of spraying or feeding first material and the second material under the drive of the drive device, and with actions of the nozzle assembly, it can achieve the effect that the medical adhesive first comes into contact with the inner wall of the in vivo duct and is coated thereon, and then the bio-ink comes into contact with the medical adhesive layer and is coated thereon. For example, it is possible that the medical adhesive is coated on the damaged part of the in vivo duct 90 during the movement of the nozzle assembly from the introducing end 20A of the catheter 20 toward the first end 90A of the to-be-repaired area 91, and then the bio-ink is coated during the movement of the nozzle assembly from the first end 90A of the to-be-repaired area 91 toward the direction of the second end 90B, and when the spraying end of the nozzle assembly returns into the catheter 20, the formation of the double-layer structure with the medical adhesive layer and the bio-ink layer is completed and the repair of the in vivo duct 90 is finished.

In the nozzle assembly 10 of the in vivo duct repair device of the above embodiments, the first material spout J1 and the second material spout J2 are arranged on a side face of the spraying end 10A of the nozzle assembly 10. At least one of the first material spout and second material spout of the nozzle assembly 10 may also be arranged on the end face of the spraying end, and by reasonably setting the positions and spraying angles of the first material spout and the second material spout, and with the movement of the nozzle assembly and the time of spraying the first material and the second material by the nozzle assembly under the drive of the drive device, the double-layer structure can be formed by spraying the medical adhesive layer and the bio-ink layer in the in vivo duct.

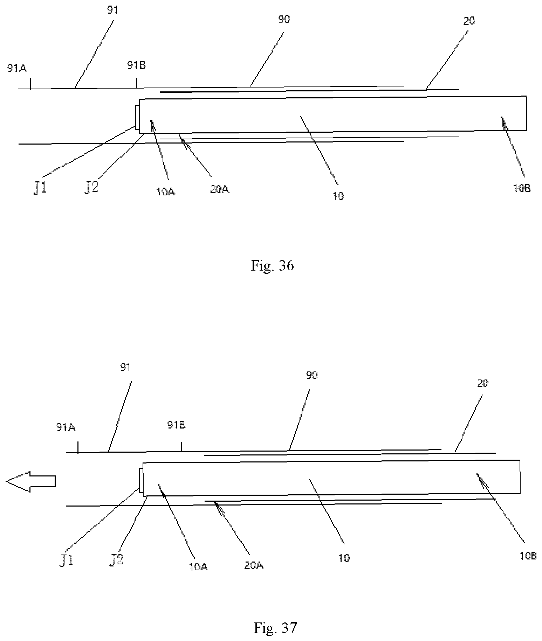

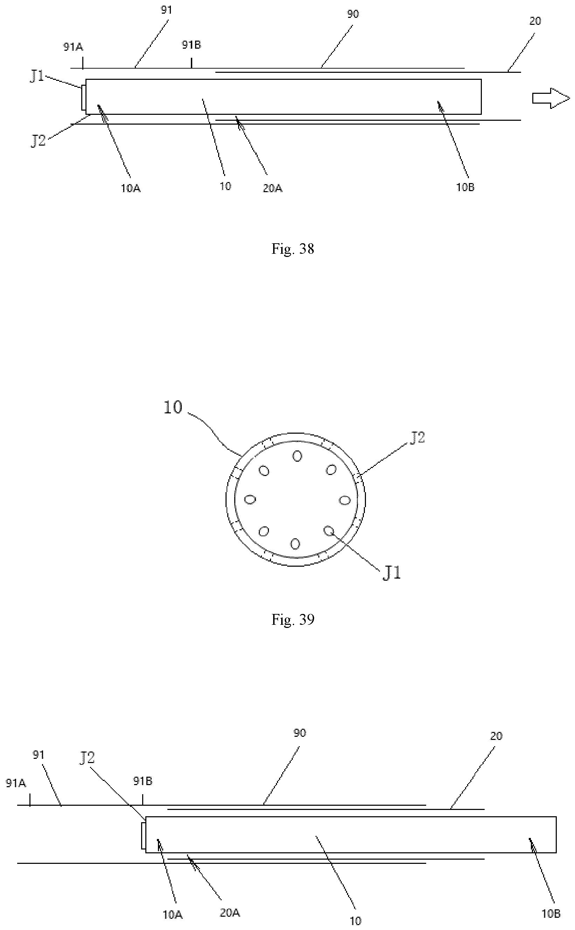

Figs. 36 to 39 show a schematic structure of an in vivo duct repair device of an embodiment of the present disclosure and an operating process thereof. Fig. 36 is a schematic structural diagram of an in vivo duct repair device of an embodiment of the present disclosure in use when the spraying end 10A of the nozzle assembly 10 is extended out of the introducing end 20A of the catheter 20 and arrives at the second end 91B of the to-be-repaired area 91 of the in vivo duct 90. Fig. 37 is a schematic structural diagram of the in vivo duct repair device of the embodiment shown in Fig. 36 in use when the spraying end 10A of the nozzle assembly 10 is extended out of the introducing end 20A of the catheter 20 and moves from the second end 91B toward the first end 91A of the to-be-repaired area 91. Fig. 38 is a schematic structural diagram of the in vivo duct repair device of the embodiment shown in Fig. 36 in use when the spraying end 10A of the nozzle assembly 10 starts moving from the first end 91A of the to-be-repaired area 91 toward the second end 91B of the to-be-repaired area 91 and at the same time starts spraying the repair materials.

In the embodiment shown in Figs. 36 to 39, the in vivo duct repair device further includes a communication pipe connected to the supplying end 10B of the nozzle assembly 10, and a drive device that is connected to the communication pipe and drives the nozzle assembly 10 to spray the first material and the second material. In some embodiments, the in vivo duct repair device may further include a guide wire. Neither the communication pipe and the drive device, nor the guide wire is shown in Figs. 36 to 39.

Fig. 39 is a schematic structural diagram of the arrangement of the first material spouts J1 and the second material spouts J2 at the spraying end 10A of the nozzle assembly 10 of the in vivo duct repair device of the embodiment shown in Fig. 36 in use. As shown in Figs. 36 to 39, the first material spouts J1 of the nozzle assembly 10 is arranged on the end face of the spraying end 10A of the nozzle assembly 10, and the second material spouts J2 is set on a side face of the spraying end 10A. The first material spouts J1 includes a plurality of circular spraying holes arranged around the circumference. The spraying directions of the circular spraying holes are inclined radially outwards with respect to the axis direction of the nozzle assembly 10 so that the first material sprayed from the first material spouts J1 can be sprayed onto the in vivo duct 90. The second material spouts J2 includes a plurality of circular spraying holes, and the plurality of spraying holes are arranged around the circumference of the nozzle assembly 10. The spraying direction of the second material spouts J2 is set radially of the nozzle assembly 10. The spraying direction of the second material spouts J2 may also be inclined radially outwards with respect to the axis direction of the nozzle assembly 10 so that the second material can be sprayed radially outwards of the nozzle assembly 10.

An operating process of the in vivo duct repair device of this embodiment is described below without providing a guide wire, and with the first material spouts J1 spraying the bio-ink and the second material spouts J2 spraying the medical adhesive as an example.

As shown in Figs. 36 to 38, to repair the in vivo duct using the vivo duct repair device of this embodiment, first, the catheter 20 is delivered to the first target position in the in vivo duct 90. The first target position may be near the second end 91B of the to-be-repaired area 91 of the in vivo duct 90 shown in Figs. 36 to 38. Then, the nozzle assembly 10 is introduced into the catheter 20 and delivered through the catheter 20 to the second target position in the in vivo duct 90. The second target position may be near the first end 91A of the to-be-repaired area 91 of the in vivo duct 90 shown in Figs. 36 to 38 where the second material spouts J2 of the nozzle assembly 10 is delivered.

When coating begins, the drive device first drives the nozzle assembly 10 to spray the medical adhesive so that the medical adhesive can be sprayed from the second material spouts J2, and at that time the nozzle assembly 10 is caused to move from the first end 91A of the to-be-repaired area 91 toward the second end 91B of the to-be-repaired area 91; in the moving process of the nozzle assembly 10, the sprayed medical adhesive comes into contact with the inner wall of the in vivo duct 90, and with the continuous movement of the nozzle assembly 10, the medical adhesive is coated on the inner wall of the in vivo duct 90, and the medical adhesive layer 91D can be formed on the inner wall of the in vivo duct 90 due to the adhering effect of the medical adhesive; when the first material spouts J1 moves to the position of the first end 91A of the to-be-repaired area 91, the drive device 40 drives the first material spouts J1 of the nozzle assembly 10 to spray the bio-ink so that the bio-ink can be sprayed from the first material spouts J1, and at that time the nozzle assembly 10 is caused to move further toward the second end 91B of the to-be-repaired area 91; in the moving process of the nozzle assembly 10, the sprayed bio-ink comes into contact with the medical adhesive layer 91D, and at the same time the bio-ink can be adhered to the medical adhesive layer 91D due to the adhering effect of the medical adhesive; and with the continuous movement of the nozzle assembly 10, the bio-ink is coated on the medical adhesive layer 91D, to form the bio-ink layer 91C on the medical adhesive layer 91D, thus ensuring the formation of the medical adhesive layer 91D and the bio-ink layer 91C on the inner wall of the in vivo duct 90, thereby achieving repair of a target area (e.g. a damaged site) in the in vivo duct 90 of a patient.

For other parts in the embodiment shown in Figs. 36 to 39 that are not described, reference may be made to relevant parts of the embodiments described above.

In the above embodiments, the first material spouts and the second material spouts of the nozzle assembly are uniformly distributed along the entire circumference of the nozzle assembly. The nozzle assembly of the above embodiments has the ability of coating the medical adhesive layer and the bio-ink layer on the entire circumference of the in vivo duct by spraying once. If local spraying is required, some of the spouts may be controlled to spray the coating materials while others of the spouts may be controlled not to participate in the coating, to achieve local coating along the circumference of the in vivo duct, so that the repair of the in vivo duct is more targeted.

In the in vivo duct repair device of some embodiments, the first material spouts and the second material spouts of the nozzle assembly may be arranged locally along the circumference of the nozzle assembly.

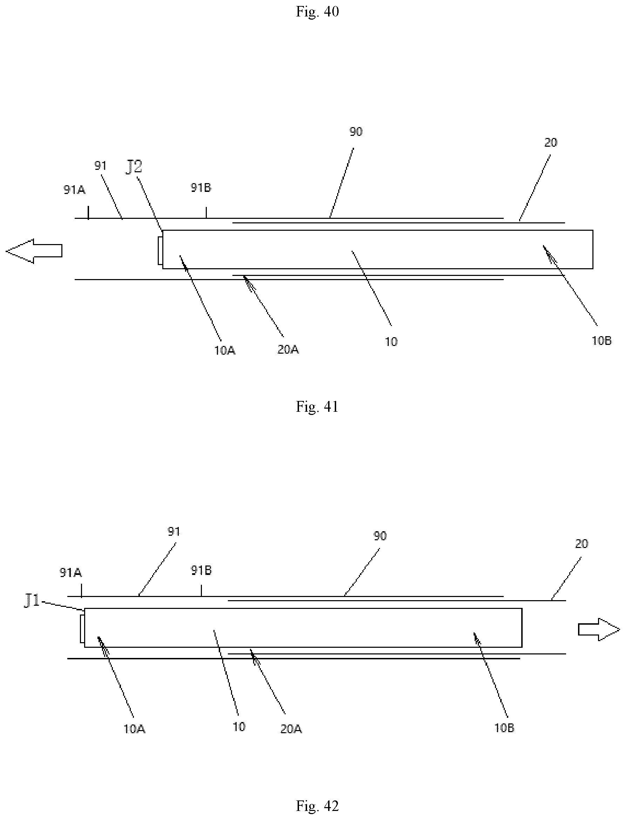

Figs. 40 to 44 show a schematic structure of an in vivo duct repair device of some embodiments of the present disclosure and an operating process thereof. Fig. 40 is a schematic structural diagram of an in vivo duct repair device of an embodiment of the present disclosure in use when the spraying end 10A of the nozzle assembly 10 is extended out of the introducing end 20A of the catheter 20 and arrives at the second end 91B of the to-be-repaired area 91 of the in vivo duct. Fig. 41 is a schematic structural diagram of the in vivo duct repair device of the embodiment shown in Fig. 40 in use when the spraying end 10A of the nozzle assembly 10 is extended out of the introducing end 20A of the catheter 20 and continues moving toward the first end 91A of the to-be-repaired area 91. Fig. 42 is a schematic structural diagram of the in vivo duct repair device of the embodiment shown in Fig. 40 in use when the spraying end 10A of the nozzle assembly 10 starts moving from the first end 91A of the to-be-repaired area 91 toward the second end 91B of the to-be-repaired area 91 and starts spraying the repair materials.

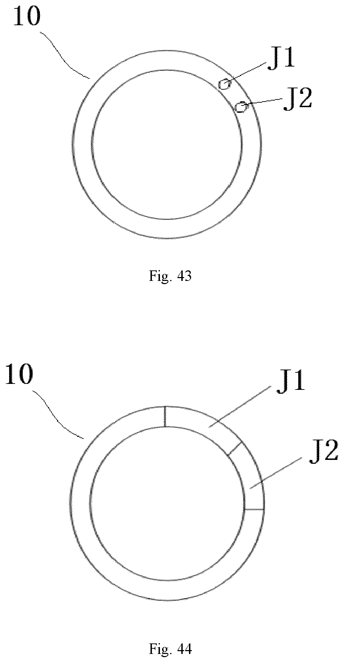

Fig. 43 is a schematic structural diagram of the arrangement of the first material spout J1 and the second material spout J2 at the spraying end 10A of a nozzle assembly 10 of the in vivo duct repair device of the embodiment shown in Fig. 40. The first material spout J1 and the second material spout J2 are both arranged on the end face of the spraying end 10A of the nozzle assembly 10. The first material spout J1 includes one circular spraying hole and the second material spout J2 includes one circular spraying hole. The first material spout J1 and the second material spout J2 are arranged around the circumference of the nozzle assembly 10.

Fig. 44 is a schematic structural diagram of the arrangement of the first material spout J1 and the second material spout J2 at the spraying end 10A of another nozzle assembly 10 of the in vivo duct repair device of the embodiment shown in Fig. 40. The first material spout J1 and the second material spout J2 of the nozzle assembly 10 shown in Fig. 44 are both arranged on the end face of the spraying end 10A of the nozzle assembly 10, and the first material spout J1 and the second material spout J2 are arranged around the circumference of the nozzle assembly 10. The nozzle assembly 10 shown in Fig. 44 differs from the nozzle assembly shown in Fig. 43 in that the first material spout J1 includes one circular-arc spraying hole and the second material spout J2 includes one circular-arc spraying hole.

In some embodiments shown in Figs. 40 to 44, the in vivo duct repair device further includes a communication pipe connected to the supplying end 10B of the nozzle assembly 10, and a drive device that is connected to the communication pipe and drives the nozzle assembly 10 to spray the first material and the second material. In some embodiments, the in vivo duct repair device may further include a guide wire. Neither the communication pipe and the drive device, nor the guide wire is shown in Figs. 40 to 42.

An operating process of the in vivo duct repair device of the embodiments shown in Figs. 40 to 44 is described below without providing a guide wire, and with the first material spout J1 spraying the bio-ink and the second material spout J2 spraying the medical adhesive as an example.

Referring to Figs. 40 to 42, the introducing end 20A of the catheter 20 is delivered to a first target position in the in vivo duct 90. The first target position may be near the second end 91B of the to-be-repaired area 91 of the in vivo duct 90 shown in Figs. 40 to 42 so that the catheter 20 forms a passage for the nozzle assembly 10 to reach the target position in the in vivo duct 90. Then the nozzle assembly 10 is introduced into the catheter 20 so that the second material spout J2 arrives at and is aligned with the second end 91B of the to-be-repaired area 91, at that time the catheter 20 and the nozzle assembly 10 are at positions as shown in Fig. 40.

The drive device 40 drives the second material spout J2 of the nozzle assembly 10 to supply the medical adhesive so that the medical adhesive can be sprayed from the second material spout J2, and then the nozzle assembly 10 is moved from the second end 91B toward the first end 91A of to-be-repaired area 91 (in the direction of an arrow as shown in Fig. 41); in the process of movement, as the medical adhesive is sprayed from the second material spout J2, the medical adhesive is coated on the inner wall of the in vivo duct 90, and the medical adhesive layer 91D is formed on the inner wall of the in vivo duct 90 due to the adhering effect of the medical adhesive; and when the nozzle assembly 10 moves to the first end 91A of the to-be-repaired area 91, the coating of the medical adhesive is completed. At that time, the nozzle assembly 10 is delivered through the catheter 20 to a second target position in the in vivo duct 90. The second target position is near the first end 91A of the to-be-repaired area 91 of the in vivo duct 90 shown in Figs. 40 to 42 where the first material spout J1 of the nozzle assembly 10 is delivered.

Then the nozzle assembly 10 is rotated so that the first material spout J1 is aligned with the medical adhesive layer, as shown in Fig. 42, and the drive device 40 drives the first material spout J1 of the nozzle assembly 10 to spray the bio-ink so that the bio-ink is sprayed from the first material spout J1, and then the nozzle assembly 10 is moved from the first end 90A towards the second end 91B of the to-be-repaired area 91 (in the direction of an arrow as shown in Fig. 42); in the process of movements, as the bio-ink is sprayed from the first material spout J1, the bio-ink is coated on the medical adhesive layer, and the bio-ink layer 91C can be formed in the medical adhesive layer due to the adhering effect of the medical adhesive; when the nozzle assembly 10 moves to the second end 91B of the to-be-repaired area 91, the coating of the bio-ink is completed, and the medical adhesive layer 91D and the bio-ink layer 91C are formed on the inner wall of the in vivo duct 90, thereby achieving repair of a target area (e.g. a damaged site) in the in vivo duct 90 of a patient.

For other parts in the embodiment shown in Figs. 40 to 44 that are not described, reference may be made to relevant parts of the embodiments described above.

Finally, it should be noted that the above embodiments are only used for describing rather than limiting the technical solutions of the present disclosure. Although the present disclosure is described in detail with reference to the preferred embodiments, those of ordinary skill in the art should understand that they still can make modifications to the specific implementations in the present disclosure or make equivalent substitutions to part of technical features thereof; and such modifications and equivalent substitutions should be encompassed within the technical solutions sought for protection in the present disclosure.

## Claims

1. An in vivo duct repair device, comprising:
a catheter (20), comprising an introducing end (20A) configured to be inserted into an in vivo duct (90);
a nozzle assembly, two axial ends of the nozzle assembly comprising a supplying end (10B) and a spraying end (10A), respectively, the nozzle assembly having a first material flow channel (P1), a first material spout (J1), a second material flow channel (P2) and a second material spout (J2), the first material flow channel (P1) being arranged inside the nozzle assembly, the first material spout (J1) being communicated with the first material flow channel (P1) and being configured to spray a first material radially outwards of the nozzle assembly, the second material flow channel (P2) being arranged inside the nozzle assembly and being isolated from the first material flow channel (P1), the second material spout (J2) being communicated with the second material flow channel (P2) and being configured to spray a second material radially outwards of the nozzle assembly, and the nozzle assembly being configured to enter the catheter (20) and extend the spraying end (10A) out of the introducing end (20A) of the catheter (20) to spray the first material and the second material to an inner wall of the in vivo duct (90); and
a drive device (40) configured to be connected to the supplying end (10B) of the nozzle assembly and configured to drive the nozzle assembly to spray the first material and the second material, wherein one of the first material and the second material is bio-ink, and the other of the first material and the second material is medical adhesive.

2. The in vivo duct repair device according to claim 1, wherein
the first material spout (J1) is located on a side face of the spraying end; and/or
the second material spout(J2) is located on a side face of the spraying end.

3. The in vivo duct repair device according to claim 1 or 2, wherein the second material spout (J2) is arranged staggeredly with respect to the first material spout (J1) in an axial direction of the nozzle assembly.

4. The in vivo duct repair device according to claim 3, wherein the first material spout (J1) is closer to an end face of the spraying end in the axial direction of the nozzle assembly than the second material spout (J2), wherein the first material spout (J1) is configured to spray the bio-ink, and the second material spout (J2) is configured to spray the medical adhesive.

5. The in vivo duct repair device according to any one of claims 1 to 4, wherein
the nozzle assembly comprises a plurality of first material spouts (J1) uniformly distributed circumferentially of the nozzle assembly; and/or
the nozzle assembly comprises a plurality of second material spouts (J2) uniformly distributed circumferentially of the nozzle assembly or the second material spout (J2) comprises an annular spout arranged around the axial direction of the nozzle assembly.

6. The in vivo duct repair device according to any one of claims 1 to 5, wherein the nozzle assembly comprises a medical adhesive scrape coating part (M) arranged on a side face of the nozzle assembly, the medical adhesive scrape coating part (M) being located between the first material spout (J1) and the second material spout (J2) in the axial direction of the nozzle assembly.

7. The in vivo duct repair device according to claim 6, wherein the medical adhesive scrape coating part (M) comprises an annular projection or brush arranged on a side face of the nozzle assembly.

8. The in vivo duct repair device according to any one of claims 1 to 7, wherein the nozzle assembly comprises:
a first nozzle body, the first material spout (J1) being arranged on the first nozzle body; and
a second nozzle body arranged at the outer periphery of the first nozzle body, the second material spout (J2) being arranged on the second nozzle body, or the second material spout (J2) being arranged between the first nozzle body and the second nozzle body.

9. The in vivo duct repair device according to claim 8, wherein
the first material flow channel (P1) is arranged in the first nozzle body; and
the second material flow channel (P2) is arranged between the second nozzle body and the first nozzle body.

10. The in vivo duct repair device according to claim 9, wherein the nozzle assembly comprises a connecting piece, which is connected between the first nozzle body and the second nozzle body.

11. The in vivo duct repair device according to any one of claims 8 to 10, wherein
the first material flow channel (P1) is arranged in the first nozzle body; and
the second material flow channel (P2) is arranged in the first nozzle body, a side wall of which has a communication port (N) communicated with the second material flow channel (P2); and
the second material spout (J2) comprises an annular spout arranged around the axial direction of the nozzle assembly, formed by the first nozzle body and an end of the second nozzle body close to the spraying end, the communication port (N) being communicated with the annular spout.

12. The in vivo duct repair device according to any one of claims 8 to 11, wherein the nozzle assembly further comprises a nozzle mounting seat, wherein
the first material flow channel (P1) comprises a first material nozzle flow channel (P11) arranged in the first nozzle body and a first material mounting seat flow channel (P12) arranged in the nozzle mounting seat, or the first material flow channel (P1) is arranged in the nozzle mounting seat; and
the second material flow channel (P2) comprises a second material nozzle flow channel (P21) arranged in the second nozzle body and a second material mounting seat flow channel (P22) arranged in the nozzle mounting seat.

13. The in vivo duct repair device according to claim 12, wherein the second material flow channel (P2) further comprises a first annular buffer groove (G), the second material nozzle flow channel (P21) and the second material mounting seat flow channel (P22) being communicated through the first annular buffer groove (G).

14. The in vivo duct repair device according to claim 13, wherein the first annular buffer groove (G) is formed between the second nozzle body and the nozzle mounting seat.

15. The in vivo duct repair device according to any one of claims 12 to 14, wherein the second material flow channel (P2) comprises a second annular buffer groove (L) arranged between the second material spout (J2) and the second material nozzle flow channel (P21).

16. The in vivo duct repair device according to any one of claims 1 to 15, wherein at least one of the first material spout (J1) and the second material spout (J2) is arranged on the end face of the spraying end (10A).

17. The in vivo duct repair device according to any one of claims 1 to 16, wherein the in vivo duct repair device further comprises a guide wire (50), the guide wire (50) being configured to obtain an initial passageway within the in vivo duct (90), and the catheter (20) is inserted along the guide wire (50) into the in vivo duct (90).

18. The in vivo duct repair device according to any of claims 1 to 16, wherein the drive device (40) comprises:
a first material supply part (41) in communication with the first material flow channel (P1) to supply the first material to the first material flow channel (P1); and/or
a second material supply part (42) in communication with the second material flow channel (P2) to supply the second material to the second material flow channel (P2).

19. The in vivo duct repair device according to claim 18, wherein
the nozzle assembly comprises a first material storage part, and the drive device (40) comprises a first drive part (41A), an outlet of the first material storage part being communicated with the first material flow channel (P1), the first drive part (41A) being coupled with the first material storage part to drive the first material storage part to output the first material from the outlet thereof; and/or
the nozzle assembly comprises a second material storage part, and the drive device (40) comprises a second drive part (41B), an outlet of the second material storage part being communicated with the second material flow channel (P2), the second drive part (41B) being coupled with the second material storage part to drive the second material storage part to output the second material from the outlet thereof.

20. The in vivo duct repair device according to claim 19, wherein
the first material storage part comprises a first bag (B1) storing the first material, and the first drive part (41A) comprises a first fluid pump; and/or
the second material storage part comprises a second bag (B2) storing the second material, and the second drive part (41B) comprises a second fluid pump.

21. The in vivo duct repair device according to claim 20, wherein
the first bag (B1) is located in the first material flow channel (P1) and separates the first material flow channel (P1) into an upstream segment and a downstream segment, the first fluid pump being communicated with the upstream segment of the first material flow channel (P1), an outlet of the first bag (B1) being communicated with the downstream segment of the first material flow channel (P1); and/or
the second bag (B2) is located in the second material flow channel (P2) and separates the second material flow channel (P2) into an upstream segment and a downstream segment, the second fluid pump being communicated with the upstream segment of the second material flow channel (P2), and an outlet of the second bag (B2) being communicated with the downstream segment of the second material flow channel (P2).

22. An operating method of the in vivo duct repair device of any one of claims 1 to 21, the method comprising:
inserting the introducing end (20A) of the catheter (20) into a to-be-repaired area (91) in the in vivo duct (90);
extending the nozzle assembly into the catheter (20) and extending the spraying end (10A) of the nozzle assembly out of the introducing end (20A) of the catheter (20); and
driving, by the drive device (40), the nozzle assembly (10) to spray the first material and the second material to the to-be-repaired area (91) to form a medical adhesive layer (91D) on the to-be-repaired area (91) and to form a bio-ink layer (91C) on the medical adhesive layer (91D).
